# EUROPEAN PATENT SPECIFICATION

(11) **EP 2 768 537 B1**
(45) Date of publication and mention of the grant of the patent: **20.02.2019**
(21) Application number: 12780812.9
(22) Date of filing: 17.10.2012
(51) Int. Cl.: A61K 47/32, A61K 9/14, A61K 9/16, A61K 9/20, A61K 31/485

(54) **ACRYLIC POLYMER FORMULATIONS**
ACRYLPOLYMERFORMULIERUNGEN
FORMULATIONS DE POLYMÈRE ACRYLIQUE

(30) Priority: 18.10.2011 US 201161548587 P
(43) Date of publication of application: 27.08.2014
(73) Proprietor: Purdue Pharma LP, Stamford, CT 06901-3431 (US)
(72) Inventor: MCKENNA, Williams, Yonkers, NY 10704 (US)
(74) Representative: Ehlich, Eva Susanne
(86) International application number: PCT/IB2012/002093
(87) International publication number: WO 2013/057570

(56) References cited:
- EP-A2- 1 138 321
- WO-A1-2005/079760
- WO-A2-01/97801
- WO-A2-2006/079550
- DE-A1- 19 617 716
- CUI ET AL: "Preparation of redispersible dry emulsion using Eudragit E100 as both solid carrier and unique emulsifier", COLLOIDS AND SURFACES. A, PHYSICACHEMICAL AND ENGINEERING ASPECTS, ELSEVIER, AMSTERDAM, NL, vol. 307, no. 1-3, 7 August 2007 (2007-08-07), pages 137-141, XP022188495, ISSN: 0927-7757, DOI: 10.1016/J.COLSURFA.2007.05.013
- EL-MALAH Y ET AL: "Novel use of Eudragit<(>R) NE 30D/Eudragit<(>R) L 30D-55 blends as functional coating materials in time-delayed drug release applications", INTERNATIONAL JOURNAL OF PHARMACEUTICS, ELSEVIER BV, NL, vol. 357, no. 1-2, 5 June 2008 (2008-06-05), pages 219-227, XP022637100, ISSN: 0378-5173, DOI: 10.1016/J.IJPHARM.2008.02.003 [retrieved on 2008-02-13]

## Description

### FIELD OF THE INVENTION

The present invention relates to the field of pharmaceutical excipients and pharmaceutical dosage forms comprising pharmaceutical excipients.

### BACKGROUND

Pharmaceutical products are sometimes the subject of abuse. For example, a particular dose of opioid agonist may be more potent when administered parenterally as compared to the same dose administered orally. Some formulations can be tampered with to provide the opioid agonist contained therein for illicit use. Opioid agonist formulations intended for oral use are sometimes crushed or subject to extraction with solvents (e.g., ethanol) by drug abusers to provide the opioid contained therein for non-prescribed illicit use (e.g., nasal or parenteral administration).

Controlled release opioid agonist dosage forms that can liberate a portion of the opioid upon exposure to ethanol can also result in a patient receiving the dose more rapidly than intended if a patient disregards instructions for use and concomitantly uses alcohol with the dosage form.

Polymers are often used in the preparation of pharmaceutical compositions. When preparing controlled release formulations, certain polymers can be intermixed with an active agent to retard the release of the active agent. Certain polymers can also be used in the preparation of pharmaceutical compositions to impart tamper resistance properties (e.g., resistance to crushing or to alcohol extraction). For example, by incorporating neutral acrylic copolymers in pharmaceutical formulations, the resultant product can exhibit rubber-like characteristics, making them more resistant to crushing. The neutral acrylic copolymer may also make a pharmaceutical formulation resistant to extraction of the active agent by solvents such as ethanol. Tamper resistance is of particular importance for products containing opioid analgesics or other active ingredients that are prone to abuse.

Neutral acrylic polymers, such as Eudragit® NE and Eudragit® NM, are manufactured such that the polymer formation occurs in a solvent and the final product is in the form of an aqueous dispersion. In the preparation of oral solid dosage forms, e.g. comprising a matrix including the neutral acrylic polymer, the aqueous dispersion of the neutral acrylic polymer as such is typically mixed with other excipients and/or with active agents in a wet granulation process.

Removing water from a pharmaceutical mixture can alter the chemical bonding among the materials in the mixture, and can slow down the formulation process by requiring an extra step of preparation to achieve a final product. The presence of excess liquid and moisture can also be problematic as many excipients and active agents are water labile which can result in a final formulation that does not have the stability required to obtain regulatory approval. Also, processing oral dosage forms comprising a matrix including the neutral acrylic polymer with an aqueous dispersion of neutral acrylic polymer places restrictions on the amount of polymer present in the dosage form/matrix. In many cases, it is difficult to achieve a dosage form/matrix containing greater than 30% by weight of neutral acrylic polymer utilizing commercially available aqueous dispersions.

There exists a need in the art for novel neutral acrylic polymer compositions and for improved processes of preparing pharmaceutical formulations, e.g. matrix formulations, based on neutral acrylic polymers. Such compositions and processes could prove useful in providing beneficial characteristics of a final product, and may allow commercially beneficial continuous processing conditions, like extrusion.

### SUMMARY OF THE INVENTION

It is an object of the present invention to provide an excipient for utilization in pharmaceutical formulations (e.g., immediate and controlled release oral solid dosage forms).

It is an object of the present invention to provide a method for preparing an excipient for utilization in pharmaceutical formulations.

It is an object of certain embodiments of the present invention to provide an oral solid dosage form comprising an active agent (e.g., an opioid analgesic), which is tamper resistant.

It is an object of certain embodiments of the present invention to provide an oral solid dosage form comprising an active agent (e.g., an opioid analgesic), which is resistant to crushing.

It is an object of certain embodiments of the present invention to provide an oral solid dosage form comprising an opioid analgesic, which is subject to less parenteral abuse than other dosage forms.

It is an object of certain embodiments of the present invention to provide an oral solid dosage form comprising an opioid analgesic, which is subject to less intranasal abuse than other dosage forms.

It is an object of certain embodiments of the present invention to provide an oral solid dosage form comprising an opioid analgesic, which is subject to less oral abuse than other dosage forms.

It is a further object of certain embodiments of the present invention to provide an oral solid dosage form comprising an opioid analgesic, which is subject to less diversion than other dosage forms.

It is a further object of certain embodiments of the present invention to provide a method of treating pain in human patients with an oral solid dosage form comprising an opioid analgesic while reducing the abuse potential of the dosage form.

It is a further object of certain embodiments of the present invention to treat a disease or condition (e.g., pain) by administering an oral solid dosage form as disclosed herein to a patient in need thereof.

It is a further object of certain embodiments of the present invention to provide a method of manufacturing an oral dosage form of an active agent (e.g., an opioid analgesic) as disclosed herein.

It is a further object of certain embodiments of the present invention to provide a use of an oral dosage form (e.g., comprising an opioid analgesic) in the manufacture of a medicament for the treatment of a disease state (e.g., pain).

The above objects of the present invention and others can be achieved by the present invention which in certain embodiments is directed to the use of purified neutral acrylic polymer as disclosed herein.

In certain embodiments, the present invention is directed to an oral solid dosage form comprising a purified neutral acrylic polymer and a prophylactically or therapeutically effective amount of an active agent.

In certain embodiments, the present invention is directed to a method of treating a condition or disease with an oral solid dosage form of the present invention, such method comprising administering an oral solid dosage form comprising purified neutral acrylic polymer and a prophylactically or therapeutically effective amount of an active agent to a patient in need thereof. For example the present invention is directed to a method of treating pain comprising administering an oral solid dosage form of the present invention to a patient in need thereof, wherein the oral solid dosage form comprises an opioid agonist.

In certain embodiments, the present invention is directed to the use of an oral solid dosage form of the present invention in the manufacture of a medicament for the treatment of pain, wherein the oral solid dosage form comprises an opioid agonist.

In certain embodiments, the present invention is directed to an oral solid dosage form of the present invention for use in the treatment of pain, wherein the oral solid dosage form comprises an opioid agonist.

In certain embodiments, the present invention is directed to a method of preparing an oral solid dosage form of the present invention comprising combining a purified neutral acrylic polymer with a prophylactically or therapeutically effective amount of an active agent (e.g., by extrusion).

In certain embodiments, the present invention is directed to a method of preparing an oral solid dosage form, comprising at least the following steps:
a) drying a dispersion (e.g., an aqueous dispersion) comprising a neutral acrylic polymer to obtain a purified neutral acrylic polymer;
b) admixing the purified neutral acrylic polymer at least with an active agent to obtain a blend, and simultaneously and/or subsequently
c) further processing the blend to obtain the oral solid dosage form.

In certain embodiments the present invention is directed to an oral solid dosage form obtainable by such a method.

In certain embodiments, the present invention is directed to a purified neutral acrylic polymer, or to a bulk powder comprising a purified neutral acrylic polymer and an active agent.

In certain embodiments, the present invention is directed to the use of a purified neutral acrylic polymer in the preparation of an oral solid dosage form, or to the use of a blend comprising a purified neutral acrylic polymer and an active agent in the preparation of an oral solid dosage form.

In describing the present invention, the following terms are to be used as indicated below. As used herein, the singular forms "a," "an," and "the" include plural references unless the context clearly indicates otherwise. Thus, for example, reference to "an active agent" includes a single active agent as well as a mixture of two or more different active agents, and reference to "a polymer" includes a single polymer as well as a mixture of two or more different polymers, and the like.

As used herein, the terms "active agent," "active ingredient," "pharmaceutical agent," and "drug" refer to any material that is intended to produce a therapeutic, prophylactic, or other intended effect, whether or not approved by a government agency for that purpose. These terms with respect to specific agents include all pharmaceutically active agents, all pharmaceutically acceptable salts thereof, and all complexes, stereoisomers, crystalline forms, cocrystals, ether, esters, hydrates and solvates thereof, and mixtures thereof.

As used herein, the term "therapeutically effective" refers to the amount of drug or the rate of drug administration needed to produce a desired therapeutic result.

As used herein, the terms "prophylactically effective" refers to the amount of drug or the rate of drug administration needed to produce a desired prophylactic result.

As used herein, the term "stereoisomers" is a general term for all isomers of individual molecules that differ only in the orientation of their atoms in space. It includes enantiomers and isomers of compounds with one or more chiral centers that are not mirror images of one another (diastereomers).

The term "enantiomer" or "enantiomeric" refers to a molecule that is nonsuperimposable on its mirror image and hence optically active wherein the enantiomer rotates the plane of polarized light in one direction and its mirror image rotates the plane of polarized light in the opposite direction.

The term "chiral center" refers to a carbon atom to which four different groups are attached.

The term "racemic" refers to a mixture of enantiomers.

The term "resolution" refers to the separation or concentration or depletion of one of the two enantiomeric forms of a molecule.

The term "patient" means a subject who has presented a clinical manifestation of a particular symptom or symptoms suggesting the need for treatment, who is treated preventatively or prophylactically for a condition, or who has been diagnosed with a condition to be treated.

The term "subject" is inclusive of the definition of the term "patient" and does not exclude individuals who are entirely normal in all respects or with respect to a particular condition.

"Pharmaceutically acceptable salts" include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate and the like; amino acid salts such as arginate, asparaginate, glutamate and the like; alkali metal salts such as sodium salt, potassium salt, cesium salt and the like; alkaline earth metal salts such as calcium salt, magnesium salt and the like; and organic amine salts such as triethylamine salt, pyridine salt, picoline salt, ethanolamine salt, triethanolamine salt, dicyclohexylamine salt, N,N'-dibenzylethylenediamine salt and the like.

The term "polyethylene oxide" is defined for purposes of the present invention as a composition of polyethylene oxide (PEO) without regard to molecular weight, and includes lower molecular weight PEOs usually referred to as polyethylene glycols. The term "high molecular weight polyethylene oxide (PEO)" is defined for purposes of the present invention as having an approximate molecular weight of at least 1,000,000, based on rheological measurements. Preferably the term "high molecular weight polyethylene oxide (PEO)" is defined for purposes of the present invention as having an approximate molecular weight of at least 1,000,000 and less than 10,000,000, based on rheological measurements. The term "low molecular weight polyethylene oxide (PEO)" is defined for purposes of the present invention as having an approximate molecular weight of less than 1,000,000, based on rheological measurements. Preferably the term "low molecular weight polyethylene oxide (PEO)" is defined for purposes of the present invention as having an approximate molecular weight of at least 1,000 and less than 1,000,000, based on rheological measurements. More preferably the term "low molecular weight polyethylene oxide (PEO)" is defined for purposes of the present invention as having an approximate molecular weight of at least 10,000 (or at least 100,000) and less than 1,000,000, based on rheological measurements. Most preferably the term "low molecular weight polyethylene oxide (PEO)" is defined for purposes of the present invention as having an approximate molecular weight of at least 10,000 (or at least 100,000) and less than 750,000, based on rheological measurements. Polyethylene oxide at the lower end of the spectrum, e.g. having an approximate molecular weight of less than 100,000, or less than 25,000, based on rheological measurements, may also be referred to as polyethylene glycol (PEG).

Polyethylene oxide is considered to have an approximate molecular weight of 1,000,000 when a 2% (by wt) aqueous solution of said polyethylene oxide using a Brookfield viscometer Model RVF, spindle No. 1, at 10 rpm, at 25°C shows a viscosity range of 400 to 800 mPa s (cP). Polyethylene oxide is considered to have an approximate molecular weight of 2,000,000 when a 2% (by wt) aqueous solution of said polyethylene oxide using a Brookfield viscometer Model RVF, spindle No. 3, at 10 rpm, at 25°C shows a viscosity range of 2000 to 4000 mPa s (cP). Polyethylene oxide is considered to have an approximate molecular weight of 4,000,000 when a 1% (by wt) aqueous solution of said polyethylene oxide using a Brookfield viscometer Model RVF, spindle No. 2, at 2 rpm, at 25°C shows a viscosity range of 1650 to 5500 mPa s (cP). Polyethylene oxide is considered to have an approximate molecular weight of 5,000,000 when a 1% (by wt) aqueous solution of said polyethylene oxide using a Brookfield viscometer Model RVF, spindle No. 2, at 2 rpm, at 25°C shows a viscosity range of 5500 to 7500 mPa s (cP). Polyethylene oxide is considered to have an approximate molecular weight of 7,000,000 when a 1% (by wt) aqueous solution of said polyethylene oxide using a Brookfield viscometer Model RVF, spindle No. 2, at 2 rpm, at 25°C shows a viscosity range of 7500 to 10,000 mPa s (cP). Polyethylene oxide is considered to have an approximate molecular weight of 8,000,000 when a 1% (by wt) aqueous solution of said polyethylene oxide using a Brookfield viscometer Model RVF, spindle No. 2, at 2 rpm, at 25°C shows a viscosity range of 10,000 to 15,000 mPa s (cP). Regarding the lower molecular weight polyethylene oxides; polyethylene oxide is considered to have an approximate molecular weight of 100,000 when a 5% (by wt) aqueous solution of said polyethylene oxide using a Brookfield viscometer Model RVT, spindle No. 1, at 50 rpm, at 25°C shows a viscosity range of 30 to 50 mPa s (cP) and polyethylene oxide is considered to have an approximate molecular weight of 900,000 when a 5% (by wt) aqueous solution of said polyethylene oxide using a Brookfield viscometer Model RVF, spindle No. 2, at 2 rpm, at 25°C shows a viscosity range of 8800 to 17,600 mPa s (cP).

The term "neutral acrylic polymer" for the purposes of the present invention refers to poly(meth)acrylates which do not contain free acid groups, amino groups or quaternary ammonium groups. In particular the term "neutral acrylic polymer" for the purposes of the present invention refers to a copolymer or homopolymer of acrylic acid (C₁-C₈) alkyl esters and/or methacrylic acid (C₁-C₈) alkyl esters. An example for a neutral acrylic polymer according to the present invention is a copolymer of ethyl acrylate and methyl methacrylate which is available as aqueous dispersions marketed under the tradenames Eudragit® NE 30 D and Eudragit® NE 40 D. In contrast, acrylic polymers marketed e.g. under the tradenames Eudragit® RL or Eudragit® RS do not fall under the definition of the term "neutral acrylic polymer" according to the present invention since they contain amounts of ammonioalkyl esters.

The term "methyl", "ethyl", "propyl", "butyl", "pentyl", "hexyl", "heptyl" or "octyl" for the purposes of the present invention refers to the respective alkyl radical(s) which may be unsubstituted or substituted. For example the alkyl radical(s) can be substituted with (C₁-C₈) alkyl groups, (C₁-C₈) alkenyl groups, (C₁-C₈) alkinyl groups, (C₁-C₈) hydroxyalkyl groups, (C₁-C₈) hydroxyalkenyl groups, (C₁-C₈) hydroxyalkinyl groups, (C₁-C₈) alkyloxy groups, (C₁-C₈) alkylcarbonyl groups, (C₁-C₈) alkyloxycarbonyl groups, (C₁-C₈) alkylcarboxy groups, hydroxy groups or keto groups. In preferred embodiments the alkyl radicals are unsubstituted. The alkyl radical(s) can be linear or branched, e.g. "butyl" is meant to comprise n-butyl, i-butyl, sec-butyl, and tert-butyl.

The term "(C₁-C₈) alkyl esters" for the purposes of the present invention refers to substituted or unsubstituted methyl, ethyl, propyl, butyl, pentyl, hexyl, heptyl and/or octyl esters. For example the substituents can be selected from (C₁-C₈) alkyl groups, (C₁-C₈) alkenyl groups, (C₁-C₈) alkinyl groups, (C₁-C₈) hydroxyalkyl groups, (C₁-C₈) hydroxyalkenyl groups, (C₁-C₈) hydroxyalkinyl groups, (C₁-C₈) alkyloxy groups, (C₁-C₈) alkylcarbonyl groups, (C₁-C₈) alkyloxycarbonyl groups, (C₁-C₈) alkylcarboxy groups, hydroxy groups or keto groups. In preferred embodiments the term "(C₁-C₈) alkyl esters" refers to unsubstituted methyl, ethyl, propyl, butyl, pentyl, hexyl, heptyl and/or octyl esters.

The term "(C₁-C₄) alkyl esters" for the purposes of the present invention refers to substituted or unsubstituted methyl, ethyl, propyl, and/or butyl esters. For example the substituents can be selected from (C₁-C₈) alkyl groups, (C₁-C₈) alkenyl groups, (C₁-C₈) alkinyl groups, (C₁-C₈) hydroxyalkyl groups, (C₁-C₈) hydroxyalkenyl groups, (C₁-C₈) hydroxyalkinyl groups, (C₁-C₈) alkyloxy groups, (C₁-C₈) alkylcarbonyl groups, (C₁-C₈) alkyloxycarbonyl groups, (C₁-C₈) alkylcarboxy groups, hydroxy groups or keto groups. In preferred embodiments the term "(C₁-C₄) alkyl esters" refers to unsubstituted methyl, ethyl, propyl, and/or butyl esters.

The term "purified neutral acrylic polymer" refers to a composition comprising the neutral acrylic polymer that has been obtained by drying a dispersion (e.g., an aqueous dispersion) comprising the neutral acrylic polymer prior to admixing with the active agent or with other excipients which are optionally used in the preparation of the oral solid dosage form (such as polymers, poloxamers, bulking agents, release modifying agents, retardants, plasticizers, stabilizers, diluents, fillers, lubricants, binders, granulating aids, colorants, flavorants, glidants, etc.), e.g. without the use of additional excipients to facilitate the drying process. Examples of drying utilized in the present invention include vacuum drying, lyophilization, pan drying, oven drying, freeze drying and evaporation. A purified neutral acrylic polymer of the present invention (i.e. the above-described composition comprising the neutral acrylic polymer) may optionally include besides the neutral acrylic polymer additional ingredients that are typically included in the manufacture of commercially available aqueous dispersions of a neutral acrylic polymer, such as emulsifiers (e.g., nonoxynol 100), residual solvents (e.g., ethanol and methanol) and unavoidable minor amounts of impurities (such as monomers of the neutral acrylic polymer). Furthermore, depending on the desired level of drying, a percentage of water may remain in the purified neutral acrylic polymer. The purified neutral acrylic polymer can be obtained and/or used in solid or semi-solid form, e.g., as a powder, film, granule, pastille or a condensed wet mass. In particular the purified neutral acrylic polymer of the present invention (i.e. the composition comprising the neutral acrylic polymer) preferably comprises less than about 20% (w/w) water, or less than about 15% (w/w) water, or less than about 10% (w/w) water, or less than about 5% (w/w) water. For example, the purified neutral acrylic polymer of the present invention may further comprise 0-8 % (w/w) of residual water and/or 0-5 %(w/w) of residual organic solvents (such as ethanol or methanol), and/or 0-3 % (w/w) of emulsifiers. The purified neutral acrylic polymer of the present invention (i.e. the composition comprising the neutral acrylic polymer) may additionally comprise 0-5 % (w/w) of further ingredients (such as further exipients or impurities originating from the manufacture of the aqueous dispersion comprising the neutral acrylic polymer). For example the purified neutral acrylic polymer of the present invention (i.e. the composition comprising the neutral acrylic polymer) comprises (or substantially consists of) 70-100 % (w/w) of neutral acrylic polymer, 0-10 % (w/w) of water, 0-5 % (w/w) of organic solvents, such as ethanol or methanol, 0-2 % (w/w) of emulsifiers, and optionally 0-5 % (w/w) of further ingredients. The above indicated amounts refer to the composition of the purified neutral acrylic polymer as obtained after drying and/or as further used for preparing a solid oral dosage form. It is well understood that the composition of the purified neutral acrylic polymer in the final solid oral dosage form can differ from the composition as originally obtained and used, e.g. due to further evaporation of water or residual solvents during the preparation of the oral solid dosage form. Hence, where the composition of the oral solid dosage form is defined by the percentage of purified neutral acrylic polymer, the respective values refer to the dry contents (solids content) of the purified neutral acrylic polymer, i.e. to the contents of the purified neutral acrylic polymer remaining in the final dosage form. In certain embodiments, the emulsifiers and/or residual solvents present in the aqueous dispersion comprising the neutral acrylic poylmer can be separated or partially separated from the neutral acrylic polymer by processes such as solvent extraction. The purified neutral acrylic polymer is obtained prior to subsequent processing with other ingredients in the manufacture of a pharmaceutical dosage form. In certain embodiments, the (aqueous) dispersion may have an amount of alcohol (e.g., methanol or ethanol) added to the dispersion prior to the purification (drying) process. The amount of added alcohol may be, e.g., less than about 30% (w/w), less than about 20% (w/w), less than about 10% (w/w), or less than about 5% (w/w) of the composition before drying.

In certain embodiments, purified neutral acrylic polymer (e.g., in the form of a film or a lyophilization product) can be milled into granules or a powder. In order to improve flowability and processing, a flow agent such as colloidal silica dioxide or magnesium stearate can be added to the granules or powder. These products can then be further processed into a pharmaceutical formulation, e.g., by extrusion or direct compression.

The term "strand" or "rod" are used interchangeably and refer to an elongated extrudate obtained by the processes of the invention.

### BRIEF DESCRIPTION OF THE DRAWINGS

**FIGURE 1** depicts a graphical view of the dissolution data for sample tablets and pellets of Example 1.
**FIGURE 2** depicts the pellets of Example 4 before and after milling. Figure 2A depicts 1mm x 1mm pellets, and Figure 2B depicts 2 mm x 2 mm pellets.
**FIGURE 3** depicts the dissolution of the pellets of Example 5 in (i) simulated gastric fluid and (ii) simulated gastric fluid and ethanol.
**FIGURE 4** depicts the tamper resistance of the pellets of Example 5 when submitted to frozen hammering at -4°C in simulated gastric fluid.

### DETAILED DESCRIPTION

The present invention addresses the above-described need in the art by providing a purified neutral acrylic polymer and an oral solid dosage form comprising the same. The present invention further provides methods of treating conditions or diseases using oral solid dosage forms of the present invention; methods of preparing oral solid dosage forms of the present invention, e.g., by extrusion; and bulk powders comprising a purified neutral acrylic polymer.

### Oral Solid Dosage Forms

The present invention provides an oral solid dosage form comprising a purified neutral acrylic polymer and a prophylactically or therapeutically effective amount of an active agent. The purified neutral acrylic polymer is prepared by drying a dispersion (e.g., an aqueous dispersion) comprising the neutral acrylic polymer prior to admixing with the active agent or with other excipients which are optionally used in the preparation of the oral solid dosage form (such as polymers, poloxamers, bulking agents, release modifying agents, retardants, plasticizers, stabilizers, diluents, fillers, lubricants, binders, granulating aids, colorants, flavorants, glidants, etc.), e.g. without the use of an additional ingredient that facilitates the drying process. An example of the use of an additional ingredient in the drying process is granulation of a neutral acrylic polymer dispersion with an excipient such as lactose. The purified neutral acrylic polymer may, however, contain other ingredients typically used in preparing commercially available aqueous dispersions of neutral acrylic polymers (e.g., Eudragit® NE 30 D and Eudragit® NE 40 D), such as emulsifiers and other ingredients added by the commercial supplier of the aqueous dispersion, as well as minor amounts of impurities resulting from the preparation of the aqueous dispersion comprising the neutral acrylic polymer (e.g. minor amounts of monomers of the neutral acrylic polymer) .

The purified neutral acrylic polymer is dried using any method that does not require the use of additional ingredients, such as drying agents. Such drying techniques utilized in the present invention include, without limitation, vacuum drying, lyophilization, pan drying, oven drying, freeze drying and evaporation. In certain embodiments, the purified neutral acrylic polymer obtained after drying may retain a certain amount of water and be in the form of a condensed wet mass. The amount of retained water may depend on the contemplated processing steps of the purified neutral acrylic polymer. For example, the purified neutral acrylic polymer in the form of a condensed wet mass may be subsequently coextruded with an active agent.

In certain embodiments, the purified neutral acrylic polymer is obtained after drying and/or further used in solid or semi-solid form. In certain embodiments, the purified neutral acrylic polymer comprises less than about 20% (w/w) water or less than about 15% (w/w) water or less than about 10% (w/w) water or less than 5% (w/w) water. In certain embodiments, the purified neutral acrylic polymer comprises less than about 3% (w/w) water. In certain embodiments, the purified neutral acrylic polymer comprises less than about 1% (w/w) water. In certain embodiments the purified neutral acrylic polymer comprises 0-8 % (w/w) of water and/or 0-5 % (w/w) of residual organic solvents (such as ethanol or methanol), and/or 0-3 % (w/w) of emulsifiers. The purified neutral acrylic polymer may additionally comprise 0-5 % (w/w) of further ingredients (such as further exipients or impurities originating from the manufacture of the aqueous dispersion comprising the neutral acrylic polymer). In certain embodiments the purified neutral acrylic polymer comprises (or preferably consists of) 70-100 % (w/w) of neutral acrylic polymer, 0-10 % (w/w) of water, 0-5 % (w/w) of organic solvents (such as ethanol or methanol), 0-2 % (w/w) of emulsifiers, and optionally 0-5 % (w/w) of further ingredients. In certain embodiments the purified neutral acrylic polymer comprises (or preferably consists of) 90-100 % (w/w) of neutral acrylic polymer, 0-5 % (w/w) of water, 0-3 % (w/w) of organic solvents (such as ethanol or methanol), 0-2 % (w/w) of emulsifiers, and optionally 0-2 % (w/w) of further ingredients. The above indicated percentages refer to the composition of the purified neutral acrylic polymer as obtained after drying and/or as used for further processing to a solid oral dosage form, and may not reflect the composition of the purified neutral acrylic polymer in the final oral solid dosage form, e.g. due to further evaporation of water or organic solvents during the preparation of the oral solid dosage form.

In certain embodiments, the purified neutral acrylic polymer is further processed prior to combining with the active agent or with other excipients. Examples of such further processing include milling, chopping, slicing or cutting the purified neutral acrylic polymer into smaller particles that are of optimum size for the preparation of the oral solid dosage form. The milling can be performed in the presence of dry ice. The purified neutral acrylic polymer may also be screened to obtain particles of a desired size.

As disclosed above, the purified neutral acrylic polymer can be derived from an aqueous dispersion comprising the neutral acrylic polymer. Such aqueous dispersions can, for example, comprise from about 20% (w/w) to about 50% (w/w) neutral acrylic polymer, or from about 30% (w/w) to about 40% (w/w) neutral acrylic polymer, or any other concentration level provided by a commercial supplier.

In certain embodiments, the oral solid dosage form of the present invention comprises an effective amount of purified neutral acrylic polymer to provide a controlled release of the active agent. The oral solid dosage form (e.g. a matrix formulation comprising at least the purified neutral acrylic polymer and an active agent) can, for example, comprise from about 10% (w/w) to about 90% (w/w) purified neutral acrylic polymer, or from about 20% (w/w) to about 80% (w/w) purified neutral acrylic polymer, or from about 30% (w/w) to about 80% (w/w) purified neutral acrylic polymer, or from about 30% (w/w) to about 70% (w/w) purified neutral acrylic polymer, or from about 40% (w/w) to about 60% (w/w) purified neutral acrylic polymer. In preferred embodiments the oral solid dosage form (e.g. a matrix formulation comprising at least the purified neutral acrylic polymer and an active agent) comprises greater than about 35% (w/w), or greater than about 40% (w/w), or greater than about 50% (w/w), or greater than about 60% (w/w) of the purified neutral acrylic polymer. In further preferred embodiments the oral solid dosage form (e.g. a matrix formulation comprising at least the purified neutral acrylic polymer and an active agent) comprises from about 35 % (w/w) to about 90% (w/w) of purified neutral acrylic polymer or from about 40 % (w/w) to about 90 % (w/w) of purified neutral acrylic polymer or from about 50 % (w/w) to about 90 % (w/w) of purified neutral acrylic polymer or from about 60 % (w/w) to about 90 % (w/w) of purified neutral acrylic polymer. The indicated percentages refer to the dry contents of the purified neutral acrylic polymer.

In certain embodiments, the neutral acrylic polymer is a poly(meth)acrylate which does not contain free acid groups, amino groups or quaternary ammonium groups. In certain embodiments the neutral acrylic polymer is a copolymer or homopolymer of acrylic acid (C₁-C₈) alkyl esters and/or methacrylic acid (C₁-C₈) alkyl esters. In certain embodiments the neutral acrylic polymer is a copolymer or homopolymer of acrylic acid (C₁-C₄) alkyl esters and/or methacrylic acid (C₁-C₄) alkyl esters.

In certain embodiments, the neutral acrylic polymer is a copolymer having the structural formula I 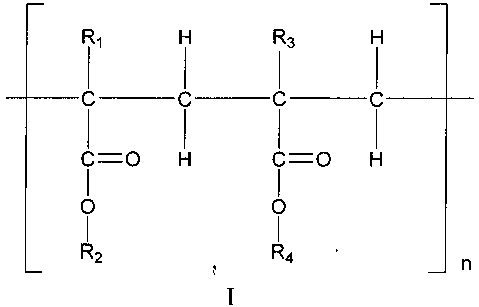
wherein R₁ and R₃ are independently selected from H and methyl,
R₂ and R₄ are independently selected from methyl, ethyl, propyl, butyl, pentyl, hexyl, heptyl or octyl,
and n is selected such that the copolymer has a mean relative molecular mass of at least about 100,000, preferably of at least about 300,000, most preferably of from about 600,000 to about 1,000,000.
In certain such embodiments, R₂ and R₄ are independently selected from methyl, ethyl, propyl, and butyl, preferably from methyl and ethyl.

In certain embodiments the neutral acrylic polymer is a copolymer of ethyl acrylate and methyl methacrylate. In certain embodiments the neutral acrylic polymer has a mean relative molecular mass of from about 600,000 to about 1,000,000, preferably of from about 600,000 to 900,000, most preferably of about 660,000, 770,000 or 800,000. In certain embodiments the neutral acrylic polymer is a copolymer of ethyl acrylate and methyl methacrylate having a mean relative molecular mass of about 800,000. Such copolymers are commercially available as aqueous dispersions marketed under the tradenames Eudragit® NE 30 D and Eudragit® NE 40 D.

The oral solid dosage form of the present invention comprises a prophylactically or therapeutically effective amount of active agent. In certain embodiments, the oral solid dosage form (e.g. a matrix formulation comprising at least the purified neutral acrylic polymer and an active agent) comprises from about 1% (w/w) to about 50% (w/w) active agent, or from about 5% (w/w) to about 40% (w/w) active agent, or from about 10% (w/w) to about 30% (w/w) active agent, or from about 15% (w/w) to about 25% (w/w) active agent. The amount of active agent present in the oral solid dosage form will vary, for example, with the type of active agent, the desired rate of release, and the condition being treated.

In addition to the purified neutral acrylic polymer, the formulations of the present invention (e.g. a matrix formulation comprising at least the purified neutral acrylic polymer and an active agent) may include additional controlled release materials (retardants). Examples of additional controlled release materials include cellulosic polymers, including but not limited to cellulose esters, cellulose diesters, cellulose triesters, cellulose ethers, cellulose ester-ethers, cellulose acylates, cellulose diacylates, cellulose triacylates, cellulose acetates, cellulose diacetates, cellulose triacetates, cellulose acetate propionates, cellulose acetate butyrates and mixtures thereof. Preferably, the cellulosic polymer is an alkyl cellulosic polymer such as ethylcellulose.

In other embodiments of the present invention, the additional controlled release material is a pharmaceutically acceptable acrylic polymer, including but not limited to acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid) (anhydride), methyl methacrylate, polymethacrylate, poly(methyl methacrylate), poly(methyl methacrylate) copolymer, polyacrylamide, aminoalkyl methacrylate copolymer, poly(methacrylic acid anhydride), glycidyl methacrylate copolymers and mixtures of any of the foregoing.

In other embodiments of the present invention, the dosage forms disclosed herein (e.g. a matrix formulation comprising at least the purified neutral acrylic polymer and an active agent) further comprise a polyethylene oxide, e.g. a high molecular weight polyethylene oxide.

The oral solid dosage form of the present invention (e.g. a matrix formulation comprising at least the purified neutral acrylic polymer and an active agent) may further comprise at least one excipient selected from the group consisting of polymers, poloxamers, bulking agents, release modifying agents, plasticizers, stabilizers, diluents, lubricants, binders, granulating aids, colorants, flavorants, and glidants. In certain embodiments, the excipient is a polymer. In certain embodiments, the excipient is polyethylene oxide. In certain embodiments, the excipient is a low molecular weight polyethylene oxide, a high molecular weight polyethylene oxide, or a mixture thereof.

In embodiments comprising a low molecular weight polyethylene oxide, the low molecular weight polyethylene oxide can, for example, have an approximate molecular weight from about 10,000 to about 750,000 Daltons, or from about 50,000 to about 500,000 Daltons, or from about 75,000 to about 300,000 Daltons, based on rheological measurements.

In embodiments comprising a high molecular weight polyethylene oxide, the high molecular weight polyethylene oxide can, for example, have an approximate molecular weight from about 1,000,000 to about 10,000,000 Daltons, or from about 2,000,000 to about 8,000,000 Daltons, or from about 4,000,000 to about 6,000,000 Daltons, based on rheological measurements.

In certain embodiments, the oral solid dosage form (e.g. a matrix formulation comprising at least the purified neutral acrylic polymer and an active agent) comprises from about 5% (w/w) to about 60% (w/w) polyethylene oxide, or from about 10% (w/w) to about 50% (w/w) polyethylene oxide, or from about 15% (w/w) to about 40% (w/w) polyethylene oxide, or from about 20% (w/w) to about 30% (w/w) polyethylene oxide. In addition to or in place of polyethylene oxide, the oral solid dosage form of the present invention (e.g. a matrix formulation comprising at least the purified neutral acrylic polymer and an active agent) can include a nonionic triblock copolymer composed of a central hydrophobic chain of polyoxypropylene (poly(propylene oxide)) flanked by two hydrophilic chains of polyoxyethylene (poly(ethylene oxide)). These compounds are commercially available under the tradenames Lutrol® and Poloxamer®.

In certain embodiments, the oral solid dosage form of the present invention (e.g. a matrix formulation comprising at least the purified neutral acrylic polymer and an active agent) comprises from about 10% (w/w) to about 90% (w/w) purified neutral acrylic polymer, from about 1% (w/w) to about 50% (w/w) active agent (e.g. oxycodone hydrochloride), and from about 5% (w/w) to about 60% (w/w) polyethylene oxide.

In certain embodiments, the oral solid dosage form of the present invention (e.g. a matrix formulation comprising at least the purified neutral acrylic polymer and an active agent) comprises from about 35% (w/w) to about 80% (w/w) purified neutral acrylic polymer, from about 5% (w/w) to about 40% (w/w) active agent (e.g. oxycodone hydrochloride), and from about 10% (w/w) to about 50% (w/w) polyethylene oxide.

In certain embodiments, the oral solid dosage form of the present invention (e.g. a matrix formulation comprising at least the purified neutral acrylic polymer and an active agent) comprises from about 50% (w/w) to about 70% (w/w) purified neutral acrylic polymer, from about 10% (w/w) to about 30% (w/w) active agent (e.g. oxycodone hydrochloride), and from about 15% (w/w) to about 40% (w/w) polyethylene oxide.

In certain embodiments, the oral solid dosage form of the present invention (e.g. a matrix formulation comprising at least the purified neutral acrylic polymer and an active agent) comprises from about 40% (w/w) to about 60% (w/w) purified neutral acrylic polymer, from about 15% (w/w) to about 25% (w/w) active agent (e.g. oxycodone hydrochloride), and from about 20% (w/w) to about 30% (w/w) polyethylene oxide.

In certain embodiments, the oral solid dosage form of the present invention (e.g. a matrix formulation comprising at least the purified neutral acrylic polymer and an active agent) comprises from about 35% (w/w) to about 90% (w/w) purified neutral acrylic polymer, from about 1% (w/w) to about 50% (w/w) active agent (e.g. oxycodone hydrochloride), and from about 5% (w/w) to about 60% (w/w) polyethylene oxide.

In certain embodiments, the oral solid dosage form of the present invention (e.g. a matrix formulation comprising at least the purified neutral acrylic polymer and an active agent) comprises from about 40% (w/w) to about 90% (w/w) purified neutral acrylic polymer, from about 5% (w/w) to about 50% (w/w) active agent (e.g. oxycodone hydrochloride), and from about 5% (w/w) to about 50% (w/w) polyethylene oxide.

In certain embodiments, the oral solid dosage form of the present invention (e.g. a matrix formulation comprising at least the purified neutral acrylic polymer and an active agent) comprises from about 50% (w/w) to about 90% (w/w) purified neutral acrylic polymer, from about 5% (w/w) to about 50% (w/w) active agent (e.g. oxycodone hydrochloride), and from about 5% (w/w) to about 40% (w/w) polyethylene oxide.

In certain embodiments, the oral solid dosage form comprises a matrix formulation comprising the purified neutral acrylic polymer and the active agent. In certain such embodiments the matrix formulation is a controlled release (extended release) matrix formulation. The matrix formulation can for example be obtained by subjecting a blend comprising the purified neutral acrylic polymer and the active agent to a direct compression step, an extrusion step, a wet granulation step, a dry granulation step, a hot molding step, or a heat compression step.

In certain such embodiments, the matrix formulation is obtained by subjecting the blend to a direct compression step, preferably by subjecting the blend to a direct compression step and a subsequent curing step. In further preferred embodiments, the matrix formulation is obtained by subjecting the blend to an extrusion step, preferably a melt extrusion step.

In certain embodiments, the oral solid dosage form comprises a matrix formulation comprising the purified neutral acrylic polymer, the active agent and a polyethylene oxide. In certain such embodiments the matrix formulation is a controlled release (extended release) matrix formulation. The matrix formulation can for example be obtained by subjecting a blend comprising the purified neutral acrylic polymer, the active agent and the polyethylene oxide to a direct compression step, an extrusion step, a wet granulation step, a dry granulation step, a hot molding step, or a heat compression step.

In certain such embodiments, the matrix formulation is obtained by subjecting the blend to a direct compression step, preferably by subjecting the blend to a direct compression step and a subsequent curing step. In further preferred embodiments, the matrix formulation is obtained by subjecting the blend to an extrusion step, preferably a melt extrusion step.

In certain embodiments, the purified neutral acrylic polymer and the active agent are in the form of an extruded blend (e.g., formed by standard extrusion or hotmelt extrusion). In further embodiments, the extruded blend further comprises polyethylene oxide. In certain embodiments, the extruded blend is in the form of a unitary dosage form that contains enough active agent for a single dose. In certain embodiments, the extruded blend is in the form of multiparticulates, such as pellets. Such pellets have, for example, a mean diameter from about 0.1 mm to about 5 mm and a mean height from about 0.1 mm to about 5 mm, or a mean diameter from about 0.5 mm to about 4 mm and a mean height from about 0.5 mm to about 4 mm, or a mean diameter from about 1 mm to about 3 mm and a mean height from about 0.5 mm to about 4 mm, or a mean diameter from about 1.5 mm to about 2.5 mm and a mean height from about 1.5 mm to about 2.5 mm. It will be appreciated by those in the art that the particular dimensions of the pellets can be varied depending on, for example, the active agent, the desired rate of release, and the specific dosage form. In certain embodiments, the pellets are in the form of spheres. The spheres have, for example, a mean diameter from about 0.1 mm to about 5 mm, or a mean diameter from about 0.5 mm to about 4 mm, or a mean diameter from about 1 mm to about 3 mm, or a mean diameter from about 1.5 mm to about 2.5 mm. In certain embodiments, the pellets are cylindrical or square. In certain embodiments, the oral solid dosage form comprises multiparticulates which are contained in a pharmaceutically acceptable capsule. In certain embodiments, the oral solid dosage form comprises multiparticulates which are compressed, e.g., into a tablet. In other embodiments, the extrudate can be injection molded into a final shape, cut from a rod or an extruded shape, or extruded into a film or slab and then punched or cut into a final shape. In other embodiments, the formulation can include a co-extrusion, wherein a coating is extruded around a core or two or more layers are extruded together.

In certain embodiments, the active agent used in the oral solid dosage form of the present invention is selected from the group consisting of ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha-two-adrenergic agonists, analgesics, anti-pyretics, anti-inflammatory agents, androgens, local and general anesthetics, anti-addictive agents, anti-androgens, anti-arrhythmic agents, antiasthmatic agents, anti-cholinergic agents, anti-cholinesterase agents, anti-coagulants, anti-diabetic agents, anti-diarrheal agents, anti-diuretic, anti-emetic and pro-kinetic agents, anti-epileptic agents, anti-estrogens, anti-fungal agents, anti-hypertensive agents, anti-microbial agents, anti-migraine agents, anti-muscarinic agents, antineoplastic agents, anti-parasitic agents, anti-parkinson's agents, anti-platelet agents, anti-progestins, anti-schizophrenia agents, anti-thyroid agents, anti-tussives, antiviral agents, atypical anti-depressants, azaspirodecanediones, barbiturates, benzodiazepines, benzothiadiazides, beta-adrenergic agonists, beta-adrenergic antagonists, selective beta-one-adrenergic antagonists, selective beta-two-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, contraceptive agents, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, hematopoietic agents, histamines, histamine antagonists, hormones, 5-hydroxytryptamine antagonists, drugs for the treatment of hyperlipoproteinemia, hypnotics, sedatives, immunosupressive agents, laxatives, methylxanthines, moncamine oxidase inhibitors, neuromuscular blocking agents, organic nitrates, opioid agonists, opioid antagonists, pancreatic enzymes, phenothiazines, progestins, prostaglandins, agents for the treatment of psychiatric disorders, retinoids, sodium channel blockers, agents for spasticity and acute muscle spasms, succinimides, testosterones, thioxanthines, thrombolytic agents, thyroid agents, tricyclic antidepressants, inhibitors of tubular transport of organic compounds, drugs affecting uterine motility, vasodilators, vitamins, and mixtures thereof, among others.

In certain embodiments, the active agent is an opioid agonist. In such embodiments, the opioid agonist is selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof. In certain embodiments, the opioid agonist is selected from the group consisting of codeine, fentanyl, hydromorphone, hydrocodone, oxycodone, dihydrocodeine, dihydromorphine, morphine, tramadol, oxymorphone, pharmaceutically acceptable salts thereof, and mixtures thereof.

In certain embodiments, the opioid agonist is selected from the group consisting of codeine, morphine, oxycodone, hydrocodone, hydromorphone, oxymorphone, tapentadol or pharmaceutically acceptable salts, hydrates and solvates thereof, and mixtures of any of the foregoing. In certain embodiments, the opioid agonist is oxycodone or a pharmaceutically acceptable salt thereof. In certain preferred embodiments, the opioid agonist is oxycodone hydrochloride.

In certain embodiments, the oral solid dosage form of the present invention comprises an active agent that is an opioid antagonist, e.g. the oral solid dosage form comprises an opioid agonist and an opioid antagonist. In such embodiments, the opioid antagonist is selected from the group consisting of amiphenazole, naltrexone, methylnaltrexone, naloxone, nalbuphine, nalorphine, nalorphine dinicotinate, nalmefene, nadide, levallorphan, cyclozocine, pharmaceutically acceptable salts thereof and mixtures thereof.

In certain embodiments, the oral solid dosage form of the present invention comprises an active agent that is a non-opioid analgesic. In such embodiments, the non-opioid analgesic is a non-steroidal anti-inflammatory agent selected from the group consisting of aspirin, celecoxib, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, pharmaceutically acceptable salts thereof and mixtures thereof.

The oral solid dosage form of the present invention may be formulated to have a certain desired release rate of active agent under certain specified conditions to provide, e.g., a 12 hour (i.e., twice-a-day) or 24 hour (i.e., once a day) formulation.

For 12 hour formulations, the dosage form can, e.g., provide a dissolution release rate in-vitro of the active agent (e.g. an opioid analgesic, such as oxycodone hydrochloride), when measured by the USP Basket Method at 100 rpm in 700 ml Simulated Gastric Fluid (SGF) at 37° C for 1 hour and thereafter switching to 900 ml Simulated Gastric Fluid (SGF) with Phosphate Buffer at a pH of 7.5 at 37° C, of at least about 15% by weight of the active agent released at 1 hour, from about 25% to about 65% by weight of the active agent released at 2 hours, from about 45% to about 85% by weight of the active agent released at 4 hours, and at least about 60% by weight of the active agent released at 8 hours.

For 24 hour formulations, the dosage form can, e.g., provide a dissolution release rate in-vitro of the active agent (e.g. an opioid analgesic, such as oxycodone hydrochloride), when measured by the USP Basket Method at 100 rpm in 700 ml Simulated Gastric Fluid (SGF) at 37° C for 1 hour and thereafter switching to 900 ml Simulated Gastric Fluid (SGF) with Phosphate Buffer at a pH of 7.5 at 37° C, of at least about 20% by weight of the active agent released at 4 hours, from about 20% to about 65% by weight of the active agent released at 8 hours, from about 45% to about 85% by weight of the active agent released at 12 hours, and at least about 80% by weight of the active agent released at 24 hours.

In certain embodiments, the dosage form is resistant to dose dumping of the active agent contained therein in the presence of alhohol. For example, in certain embodiments comprising an opioid agonist (such as oxycodone hydrochoride), the amount of opioid agonist released at 1 hour in 900 mL 0.1 N HCl (pH 1.5) with 40% EtOH using USP Apparatus II at 50 rpm is not more than the amount of opioid agonist released at 1 hour in 900 mL 0.1 N HCl (pH 1.5) without EtOH using USP Apparatus II at 50 rpm.

In certain embodiments, the amount of opioid agonist (e.g. oxycodone hydrochoride) released at 1 hour in 900 mL 0.1 N HCl (pH 1.5) with 40% EtOH using USP Apparatus II at 50 rpm is less than the amount of opioid agonist released at 1 hour in 900 mL 0.1 N HCl (pH 1.5) without EtOH using USP Apparatus II at 50 rpm.

In certain embodiments, the amount of opioid agonist (e.g. oxycodone hydrochoride) released at 1 hour in 900 mL 0.1 N HCl (pH 1.5) with 40% EtOH using USP Apparatus II at 50 rpm is within 25% (w/w) of the amount of opioid agonist released at 1 hour in 900 mL 0.1 N HCl (pH 1.5) without EtOH using USP Apparatus II at 50 rpm.

In certain embodiments, the amount of opioid agonist (e.g. oxycodone hydrochoride) released at 1 hour in 900 mL 0.1 N HCl (pH 1.5) with 40% EtOH using USP Apparatus II at 50 rpm is within 10% (w/w) of the amount of opioid agonist released at 1 hour in 900 mL 0.1 N HCl (pH 1.5) without EtOH using USP Apparatus II at 50 rpm.

In certain embodiments, the amount of opioid agonist (e.g. oxycodone hydrochoride) released at 2 hour in 900 mL 0.1 N HCl (pH 1.5) with 40% EtOH using USP Apparatus II at 50 rpm is not more than the amount of opioid agonist released at 2 hour in 900 mL 0.1 N HCl (pH 1.5) without EtOH using USP Apparatus II at 50 rpm.

In certain embodiments, the amount of opioid agonist (e.g. oxycodone hydrochoride) released at 2 hour in 900 mL 0.1 N HCl (pH 1.5) with 40% EtOH using USP Apparatus II at 50 rpm is less than the amount of opioid agonist released at 2 hour in 900 mL 0.1 N HCl (pH 1.5) without EtOH using USP Apparatus II at 50 rpm.

In certain embodiments, the amount of opioid agonist (e.g. oxycodone hydrochoride) released at 2 hour in 900 mL 0.1 N HCl (pH 1.5) with 40% EtOH using USP Apparatus II at 50 rpm is within 25% (w/w) of the amount of opioid agonist released at 2 hour in 900 mL 0.1 N HCl (pH 1.5) without EtOH using USP Apparatus II at 50 rpm.

In certain embodiments, the amount of opioid agonist (e.g. oxycodone hydrochoride) released at 2 hour in 900 mL 0.1 N HCl (pH 1.5) with 40% EtOH using USP Apparatus II at 50 rpm is within 10% (w/w) of the amount of opioid agonist released at 2 hour in 900 mL 0.1 N HCl (pH 1.5) without EtOH using USP Apparatus II at 50 rpm.

In certain embodiments, the amount of opioid agonist (e.g. oxycodone hydrochoride) released at 4 hour in 900 mL 0.1 N HCl (pH 1.5) with 40% EtOH using USP Apparatus II at 50 rpm is not more than the amount of opioid agonist released at 4 hour in 900 mL 0.1 N HCl (pH 1.5) without EtOH using USP Apparatus II at 50 rpm.

In certain embodiments, the amount of opioid agonist (e.g. oxycodone hydrochoride) released at 4 hour in 900 mL 0.1 N HCl (pH 1.5) with 40% EtOH using USP Apparatus II at 50 rpm is less than the amount of opioid agonist released at 4 hour in 900 mL 0.1 N HCl (pH 1.5) without EtOH using USP Apparatus II at 50 rpm.

In certain embodiments, the amount of opioid agonist (e.g. oxycodone hydrochoride) released at 4 hour in 900 mL 0.1 N HCl (pH 1.5) with 40% EtOH using USP Apparatus II at 50 rpm is within 25% (w/w) of the amount of opioid agonist released at 4 hour in 900 mL 0.1 N HCl (pH 1.5) without EtOH using USP Apparatus II at 50 rpm.

In certain embodiments, the amount of opioid agonist (e.g. oxycodone hydrochoride) released at 4 hour in 900 mL 0.1 N HCl (pH 1.5) with 40% EtOH using USP Apparatus II at 50 rpm is within 10% (w/w) of the amount of opioid agonist released at 4 hour in 900 mL 0.1 N HCl (pH 1.5) without EtOH using USP Apparatus II at 50 rpm.

In certain embodiments, the amount of opioid agonist (e.g. oxycodone hydrochoride) released at 1 hour is from about 10% (w/w) to about 30% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 2 hours is from about 25% (w/w) to about 50% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 4 hours is from about 40% (w/w) to about 80% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 8 hours is from about 65% (w/w) to about 95% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 12 hours is greater than about 80% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 1 hour is from about 15% (w/w) to about 25% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 2 hours is from about 30% (w/w) to about 40% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 4 hours is from about 55% (w/w) to about 75% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 8 hours is from about 75% (w/w) to about 85% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 12 hours is greater than about 90% (w/w) as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 1 hour is from about 10% (w/w) to about 30% (w/w); the amount of opioid agonist released at 2 hours is from about 25% (w/w) to about 50% (w/w); the amount of opioid agonist released at 4 hours is from about 40% (w/w) to about 80% (w/w); the amount of opioid agonist released at 8 hours is from about 65% (w/w) to about 95% (w/w), and the amount of opioid agonist released at 12 hours is greater than about 80% (w/w); as measured by an in-vitro dissolution in a USP Apparatus 1 (basket) at 100 rpm in 900 ml simulated gastric fluid without enzymes (SGF) at 37° C.

In certain embodiments, the amount of opioid agonist (e.g. oxycodone hydrochoride) released at 1 hour is from about 15% (w/w) to about 25% (w/w); the amount of opioid agonist released at 2 hours is from about 30% (w/w) to about 40% (w/w); the amount of opioid agonist released at 4 hours is from about 55% (w/w) to about 75% (w/w); the amount of opioid agonist released at 8 hours is from about 75% (w/w) to about 85% (w/w), and the amount of opioid agonist released at 12 hours is greater than about 90% (w/w); as measured by an in-vitro dissolution in a USP Apparatus 1 (basket) at 100 rpm in 900 ml simulated gastric fluid without enzymes (SGF) at 37° C.

In certain embodiments, the oral solid dosage form of the present invention demonstrates the tamper-resistant characteristic of not breaking or shattering when force is applied to it (by, for example, striking it with a hammer). Instead, the oral solid dosage form flattens without breaking or shattering. This characteristic makes it more difficult for the oral solid dosage form to be abused, by snorting the powder of a shattered tablet, chewing a tablet, or injecting a solution prepared from a shattered tablet.

In certain embodiments, the oral solid dosage form can be flattened without breaking, wherein the thickness of the dosage form after flattening corresponds to no more than about 60% of the thickness of the dosage form before flattening.

In certain embodiments, the dosage form can be flattened without breaking, wherein the thickness of the dosage form after flattening corresponds to no more than about 50% of the thickness of the dosage form before flattening.

In certain embodiments, the dosage form can be flattened without breaking, wherein the thickness of the dosage form after flattening corresponds to no more than about 40% of the thickness of the dosage form before flattening.

In certain embodiments, the dosage form can be flattened without breaking, wherein the thickness of the dosage form after flattening corresponds to no more than about 30% of the thickness of the dosage form before flattening.

In certain embodiments, the dosage form can be flattened without breaking, wherein the thickness of the dosage form after flattening corresponds to no more than about 20% of the thickness of the dosage form before flattening.

In certain embodiments, the amount of opioid agonist released at 0.5 hour from a flattened dosage form deviates no more than about 20% points from a non-flattened dosage form as measured by an in-vitro dissolution in a USP Apparatus 1 (basket) at 100 rpm in 900 ml simulated gastric fluid without enzymes (SGF) at 37° C.

In certain embodiments, the amount of opioid agonist released at 0.5 hour from a flattened dosage form deviates no more than about 15% points from a non-flattened dosage form as measured by an in-vitro dissolution in a USP Apparatus 1 (basket) at 100 rpm in 900 ml simulated gastric fluid without enzymes (SGF) at 37° C.

In certain embodiments, the amount of opioid agonist released at 0.5 hour from a flattened dosage form deviates no more than about 10% points from a non-flattened dosage form as measured by an in-vitro dissolution in a USP Apparatus 1 (basket) at 100 rpm in 900 ml simulated gastric fluid without enzymes (SGF) at 37° C.

In certain embodiments the oral solid dosage form according to the present invention is prepared by the method of preparing as described further below.

The invention also relates to an oral solid dosage form obtainable by the method of preparing as described further below.

### Methods of Treatment

The present invention is further directed to a method of treating a disease or condition comprising administering any of the oral solid dosage forms described herein to a patient in need thereof. In certain embodiments, the patient is treated for pain, diarrhea, or constipation. In certain embodiments, wherein the oral solid dosage form comprises an opioid analgesic (e.g. oxycodone hydrochloride), the patient is treated for pain.

The method of treatment of the present invention may comprise administering the oral solid dosage form described herein with another pharmaceutical composition. In certain embodiments, the other pharmaceutical composition is administered to treat the same condition or disease. In other embodiments, the other pharmaceutical composition is administered to treat a different condition or disease.

In certain embodiments, the method of treatment of the present invention further comprises monitoring the patient for how the patient metabolizes the active agent, or how the patient responds to the active agent. In certain embodiments, the method of treatment further comprises altering the dose of the oral solid dosage form in response to said monitoring. In certain embodiments, certain baseline measurements are taken prior to administering the oral solid dosage form to the patient.

### Methods of Preparation

The present invention is further directed to a method of preparing an oral solid dosage form as disclosed herein. In certain embodiments the method comprises at least the following steps:
a) drying a dispersion (e.g., an aqueous dispersion) comprising a neutral acrylic polymer to obtain a purified neutral acrylic polymer;
b) admixing the purified neutral acrylic polymer at least with an active agent to obtain a blend, and simultaneously and/or subsequently
c) further processing the blend to obtain the oral solid dosage form.

In certain such embodiments, the drying is performed by vacuum drying, lyophilization, pan drying, oven drying, freeze drying and/or evaporation. Preferably the drying is performed by vacuum drying or oven drying. In one preferred embodiment the drying is performed by vacuum drying. In another preferred embodiment the drying is performed by oven drying.

Depending on the desired level of drying, a percentage of water may remain in the purified neutral acrylic polymer. In certain embodiments the purified neutral acrylic polymer (as obtained in step a) by drying and/or as further used in step b)) can be in solid or semi-solid form, e.g., in form of a powder, film, granule, pastille or a condensed wet mass.

In preferred embodiments the purified neutral acrylic polymer obtained in step a) by drying the aqueous dispersion comprising the neutral acrylic polymer comprises less than about 20% (w/w) water or less than about 15% (w/w) water, preferably less than about 10% (w/w) water or less than about 5% (w/w) water, more preferably less than about 3% (w/w) water, and most preferably less than about 1% (w/w) water. For example the purified neutral acrylic polymer of the present invention may comprise 0-8 % (w/w) of residual water and/or 0-5 %(w/w) of residual organic solvents (such as ethanol or methanol), and/or 0-3 % (w/w) of emulsifiers.

Depending on the conditions of drying, the purified neutral acrylic polymer obtained after drying can be e.g. in the form of sheets, and it may be appropriate to reduce the size of the purified neutral acrylic polymer before admixing it in step b) with the active agent and optionally with other excipients of the oral solid dosage form. Hence in step a), the purified neutral acrylic polymer obtained by drying the aqueous dispersion may subsequently be milled. In certain embodiments the milling procedure is preceded by cutting, slicing or breaking the purified neutral acrylic polymer.

In certain embodiments the milling is conducted in the presence of dry ice.

In certain embodiments wherein step a) comprises a drying and a subsequent milling step, the purified neutral acrylic polymer is screened after being milled. For example the purified neutral acrylic polymer may be passed through a U.S. mesh screen of appropriate size, e.g. through a #14 U.S. mesh screen or through a #18 U.S. mesh screen.

In step b), the purified neutral acrylic polymer which is optionally milled or milled and screened, can, in addition to the active agent, be further admixed with at least one excipient selected from the group consisting of polymers, poloxamers, bulking agents, release modifying agents, plasticizers, stabilizers, diluents, lubricants, binders, granulating aids, colorants, flavorants, and glidants.

In certain embodiments the purified neutral acrylic polymer is in step b) further admixed with a polymer. In certain such embodiments the polymer is a polyethylene oxide. The polyethylene oxide can be a high molecular weight polyethylene oxide, a low molecular weight polyethylene oxide, or a mixture thereof. Preferably the polyethylene oxide is a low molecular weight polyethylene oxide. For example the polyethylene oxide has an approximate molecular weight of from about 10,000 Daltons to about 750,000 Daltons, based on rheological measurements, preferably an approximate molecular weight of from about 50,000 Daltons to about 500,000 Daltons, based on rheological measurements, and most preferably an approximate molecular weight of from about 75,000 Daltons to about 300,000 Daltons, based on rheological measurements.

The amounts of purified neutral acrylic polymer, active agent and optional further ingredients (e.g. polyethylene oxide) to be admixed in step b) are preferably selected such that the above-described compositional features (percentages) of the oral solid dosage form are achieved.

The oral solid dosage form obtained in step c) can be a unitary dosage form, such as a tablet. Alternatively the oral solid dosage form is in the form of multiparticulates (e.g. pellets or spheres) which are e.g. filled into a capsule or compressed into a tablet.

In preferred embodiments the oral solid dosage form comprises a matrix formulation comprising the purified neutral acrylic polymer and the active agent. Most preferably the matrix formulation is a controlled release matrix formulation.

In certain embodiments, the blend is further processed in step c) by subjecting it to a direct compression step, an extrusion step, a wet granulation step, a dry granulation step, a hot molding step, or a heat compression step.

In one embodiment the blend is further processed in step c) by subjecting it to a direct compression step, yielding either a unitary oral solid dosage form in the form of a tablet, or multiparticulates.

In certain embodiments the blend is further processed in step c) by subjecting it to a direct compression step and a subsequent curing step. The conditions of the curing step depend inter alia on the amounts of purified neutral acrylic polymer and optional polyethylene oxide present in the oral solid dosage form. Suitable conditions are described further below.

In certain preferred embodiments the blend is further processed in step c) by subjecting it to an extrusion step. The extrusion step can be a melt-extrusion step (e.g., at a temperature from about 100°C to about 120°C). The resulting extrudate can subsequently be divided into unitary dosage forms, preferably in the form of a tablet. Alternatively the extrudate obtained in step c) is subsequently divided into multiparticulates, preferably in the form of pellets or spheres. The multiparticulates can be filled into a capsule, or can be compressed (e.g. with other excipients such as fillers or binders) into a tablet. In certain such embodiments the extrudate is not allowed to cool before it is divided.

In certain embodiments, the method comprises at least the following steps: (i) mixing (e.g. in an extruder) the purified neutral acrylic polymer and the active agent; (ii) extruding the mixture as a strand; (iii) optionally cooling the strand; and (iv) dividing the strand into unit doses. The divided unit dose can be in the form of a unitary tablet (molded or non-molded) or can be in the form of multiparticulates that are subsequently compressed to a tablet or contained in a capsule. In certain embodiments, the method comprises at least the following steps: (i) mixing (e.g. in an extruder) the purified neutral acrylic polymer, the active agent; and polyethylene oxide (ii) extruding the mixture as a strand; (iii) optionally cooling the strand; and (iv) dividing the strand into unit doses. As explained above, oral solid dosage forms according to the present invention using the purified neutral acrylic polymer described herein may further be prepared by processes other than extrusion. For example, the ingredients can be blended and directly compressed or the ingredients can be wet or dry granulated and subsequently compressed or contained in capsules.

In embodiments involving extrusion, the shape of the extruded strand can be varied, e.g., by changing the shape of the opening out of which the strand is extruded or changing the length of each individually divided strand. Varied strand shapes will yield varied pellet shapes after the extruded strand is divided which may confer benefits depending on the type of active agent and the specific dosage form. The extruded strand may be cooled at room temperature, or at a temperature cooler than room temperature. The extruded strand may also be cooled in a step-wise fashion at different temperatures for specified amounts of time after the strand is extruded. Controlling the rate and temperature at which a strand cools may confer a particular shape (which may affect the dissolution profile) upon the cooled strand. In certain embodiments, it may be advantageous to divide the strand after very little or no cooling; as the divided pellets cool, they may expand (or contract), thereby taking on a nearly spherical shape.

The preparation of oral solid dosage forms can also include the incorporation of additional pharmaceutically acceptable components, e.g., lubricants, binders, granulating aids, diluents, colorants, flavorants (e.g., bittering agents) and glidants.

### BULK POWDERS

The present invention is further directed to a bulk powder comprising a purified neutral acrylic polymer and an active agent. In certain embodiments, the purified neutral acrylic polymer has been dried by vacuum drying, lyophilization, pan drying, freeze drying or oven drying. The bulk powder of the present invention may, for example, be used to prepare the oral solid dosage forms described herein. The bulk powder of the present invention may, for example, be used in the method of preparation described herein, including, for example, extrusion.

### CURED FORMULATIONS

In certain embodiments, in particular in certain embodiments wherein the oral solid dosage form is prepared by direct compression of a blend comprising a purified neutral acrylic polymer and an active agent and optionally polyethylene oxide, a the method of the present invention may further comprise in step c) the step of curing the final dosage form. Curing is a process wherein the dosage form is subjected to certain conditions such as heat or electromagnetic radiation for a specified time in order to obtain a functional or physical change in the dosage form. The functional change can be the dosage form exhibiting a dissolution profile that does not change substantially over time. The physical change can be the hardening of certain polymers (e.g., polyethylene oxides) or a stable dissolution profile provided by certain polymers (e.g., neutral acrylic polymers) that may be included in the dosage form.

For embodiments comprising polyethylene oxide in a controlled release formulation, the curing step may comprise at least partially melting the polyethylene oxide in the formulation. In certain embodiments, at least about 20% or at least about 30% of the polyethylene oxide in the formulation melts. Preferably, at least about 40%, or at least about 50%, or at least about 60%, or at least about 75%, or at least about 90% of the polyethylene oxide in the formulation melts during the curing step. In a preferred embodiment, about 100% of the polyethylene oxide melts.

In other embodiments, the curing step comprises subjecting the formulation to an elevated temperature for a certain period of time. In such embodiments, the curing temperature is at least as high as the softening temperature of the polyethylene oxide. According to certain embodiments, the curing temperature is at least about 60°C, at least about 62°C, ranges from about 62°C to about 90°C, from about 62°C to about 85°C, from about 62°C to about 80°C, from about 65°C to about 90°C, from about 65°C to about 85°C, or from about 65°C to about 80°C. The curing temperature preferably ranges from about 68°C to about 90°C, from about 68°C to about 85°C, from about 68°C to about 80°C, from about 70°C to about 90°C, from about 70°C to about 85°C, from about 70°C to about 80°C, from about 72°C to about 90°C, from about 72°C to about 85°C or from about 72°C to about 80°C. The curing temperature may be at least about 60°C, at least about 62°C, less than about 90°C or less than about 80°C. Preferably, it is in the range of from about 62°C to about 72°C or from about 68°C to about 72°C. Preferably, the curing temperature is at least as high as the lower limit of the softening temperature range of the polyethylene oxide, or at least about 62°C, or at least about 68°C. In further embodiments, the curing temperature is at least as high as the upper limit of the softening temperature range of the polyethylene oxide, or at least about 72°C. In further embodiments, the curing temperature is higher than the upper limit of the softening temperature range of the polyethylene oxide, or at least about 75°C, or at least about 80°C.

In those embodiments where the curing step involves subjecting the formulation to an elevated temperature for a certain period of time, this period of time is hereinafter referred to as the curing time. For the measurement of the curing time, a starting point and an end point of the curing step are defined. For the purposes of the present invention, the starting point of the curing step is defined to be the point in time when the curing temperature is reached.

In certain embodiments, the temperature profile during the curing step shows a plateau-like form between the starting point and the end point of the curing. In such embodiments, the end point of the curing step is defined to be the point in time when the heating is stopped or at least reduced, e.g. by terminating or reducing the heating and/or by starting a subsequent cooling step, and the temperature subsequently drops below the curing temperature by more than about 10°C and/or below the lower limit of the softening temperature range of polyethylene oxide, for example, below about 62°C. When the curing temperature is reached and the curing step is thus started, deviations from the curing temperature in the course of the curing step can occur. Such deviations are tolerated as long as they do not exceed a value of about ± 10°C, preferably about ± 6°C, and more preferably about ± 3°C. For example, if a curing temperature of at least about 75°C is to be maintained, the measured temperature may temporarily increase to a value of about 85°C, about 81°C, or about 78°C, and the measured temperature may also temporarily drop down to a value of about 65°C, about 69°C or about 72°C. In the cases of a larger decrease of the temperature and/or in the case that the temperature drops below the lower limit of the softening temperature range of polyethylene oxide, for example below about 62°C, the curing step is discontinued, i.e. an end point is reached. Curing can be restarted by again reaching the curing temperature.

In other embodiments, the temperature profile during the curing step shows a parabolic or triangular form between the starting point and the end point of the curing. This means that after the starting point, i.e., the point in time when the curing temperature is reached, the temperature further increases to reach a maximum, and then decreases. In such embodiments, the end point of the curing step is defined to be the point in time when the temperature drops below the curing temperature.

Depending on the apparatus used for the curing (i.e., curing device), different temperatures within the curing device can be measured to characterize the curing temperature.

In certain embodiments, the curing step may take place in an oven. In such embodiments, the temperature inside the oven is measured. Based thereon, when the curing step takes place in an oven, the curing temperature is defined to be the target inside temperature of the oven and the starting point of the curing step is defined to be the point in time when the inside temperature of the oven reaches the curing temperature. The end point of the curing step is defined to be (1) the point in time when the heating is stopped or at least reduced and the temperature inside the oven subsequently drops below the curing temperature by more than about 10°C and/or below the lower limit of the softening temperature range of the polyethylene oxide , for example below about 62°C, in a plateau-like temperature profile or (2) the point in time when the temperature inside the oven drops below the curing temperature in a parabolic or triangular temperature profile. Preferably, the curing step starts when the temperature inside the oven reaches a curing temperature of at least about 62°C, at least about 68°C, at least about 70°C, at least about 72°C or at least about 75°C. In preferred embodiments, the temperature profile during the curing step shows a plateau-like form, wherein the curing temperature, i.e. the inside temperature of the oven, is at least about 68°C, about 70°C, about 72°C, about 73°C, or lies within a range of from about 70°C to about 75°C, and the curing time is preferably in the range of from about 30 minutes to about 20 hours, from about 30 minutes to about 15 hours, from about 30 minutes to about 4 hours, or from about 30 minutes to about 2 hours. In certain embodiments, the curing time is in the range of from about 30 minutes to about 90 minutes.

In certain other embodiments, the curing takes place in curing devices that are heated by an air flow and comprise a heated air supply (inlet) and an exhaust, e.g., a coating pan or fluidized bed. Such curing devices will hereinafter be called convection curing devices. In such curing devices, it is possible to measure the temperature of the inlet air, i.e., the temperature of the heated air entering the convection curing device and/or the temperature of the exhaust air, i.e., the temperature of the air leaving the convection curing device. It is also possible to determine or at least estimate the temperature of the formulations inside the convection curing device during the curing step, e.g., by using infrared temperature measurement instruments (such as an IR gun) or by measuring the temperature using a temperature probe that was placed inside the curing device near the formulations. Based thereon, when the curing step takes place in a convection curing device, the curing temperature can be defined and the curing time can be measured as follows.

In one embodiment (method 1), the curing temperature is defined to be the target inlet air temperature and the starting point of the curing step is defined to be the point in time when the inlet air temperature reaches the curing temperature. The end point of the curing step is defined to be (1) the point in time when the heating is stopped or at least reduced and the inlet air temperature subsequently drops below the curing temperature by more than about 10°C and/or below the lower limit of the softening temperature range of the polyethylene oxide, for example below about 62°C, in a plateau-like temperature profile, or (2) the point in time when the inlet air temperature drops below the curing temperature in a parabolic or triangular temperature profile. Preferably, the curing step starts according to method 1, when the inlet air temperature reaches a curing temperature of at least about 62°C, at least about 68°C, at least about 70°C, at least about 72°C or at least about 75°C. In a preferred embodiment, the temperature profile during the curing step shows a plateau-like form, wherein the curing temperature, i.e. the target inlet air temperature, is preferably at least about 72°C, for example, about 75°C, and the curing time which is measured according to method 1 is preferably in the range of from about 15 minutes to about 2 hours, for example, about 30 minutes or about 1 hour.

In another embodiment (method 2), the curing temperature is defined to be the target exhaust air temperature, and the starting point of the curing step is defined to be the point in time when the exhaust air temperature reaches the curing temperature. The end point of the curing step is defined to be (1) the point in time when the heating is stopped or at least reduced and the exhaust air temperature subsequently drops below the curing temperature by more than about 10°C and/or below the lower limit of the softening temperature range of the polyethylene oxide, for example below about 62°C, in a plateau-like temperature profile, or (2) the point in time when the exhaust air temperature drops below the curing temperature in a parabolic or triangular temperature profile. Preferably, the curing step starts according to method 2, when the exhaust air temperature reaches a curing temperature of at least about 62°C, at least about 68°C, at least about 70°C, at least about 72°C or at least about 75°C. In preferred embodiments, the temperature profile during the curing step shows a plateau-like form, wherein the curing temperature, i.e. the target exhaust air temperature, is preferably at least about 68°C, at least about 70°C or at least about 72°C, for example the target exhaust air temperature is about 68°C, about 70°C, about 72°C, about 75°C or about 78°C, and the curing time which is measured according to method 2 is preferably in the range of from about 1 minute to about 2 hours or from about 5 minutes to about 90 minutes, for example, the curing time is about 5 minutes, about 10 minutes, about 15 minutes, about 30 minutes, about 60 minutes, about 70 minutes, about 75 minutes or about 90 minutes. In a more preferred embodiment, the curing time which is measured according to method 2 is in the range of from about 15 minutes to about 1 hour.

In a further embodiment (method 3), the curing temperature is defined to be the target temperature of the formulations and the starting point of the curing step is defined to be the point in time when the temperature of the formulations, which can be measured for example by an IR gun, reaches the curing temperature. The end point of the curing step is defined to be (1) the point in time when the heating is stopped or at least reduced and the temperature of the formulations subsequently drops below the curing temperature by more than about 10°C and/or below the lower limit of the softening temperature range of the polyethylene oxide, for example below about 62°C, in a plateau-like temperature profile or (2) the point in time when the temperature of the formulations drops below the curing temperature in a parabolic or triangular temperature profile. Preferably, the curing step starts according to method 3, when the temperature of the formulations reaches a curing temperature of at least about 62°C, at least about 68°C, at least about 70°C, at least about 72°C or at least about 75°C.

In still another embodiment (method 4), the curing temperature is defined to be the target temperature measured using a temperature probe, such as a wire thermocouple, that is placed inside the curing device near the formulations, and the starting point of the curing step is defined to be the point in time when the temperature measured using the temperature probe reaches the curing temperature. The end point of the curing step is defined to be (1) the point in time when the heating is stopped or at least reduced and the temperature measured using the temperature probe subsequently drops below the curing temperature by more than about 10°C and/or below the lower limit of the softening temperature range of polyethylene oxide, for example below about 62°C, in a plateau-like temperature profile, or (2) the point in time when the temperature measured using the temperature probe drops below the curing temperature in a parabolic or triangular temperature profile. Preferably, the curing step starts when the temperature measured using a temperature probe registers a temperature in the curing device of at least about 62°C, at least about 68°C, at least about 70°C, at least about 72°C or at least about 75°C. In a preferred embodiment, the temperature profile during the curing step shows a plateau-like form, wherein the curing temperature is at least about 68°C, for example, about 70°C, and the curing time which is measured according to method 4 is preferably in the range of from about 15 minutes to about 2 hours or about 60 minutes or about 90 minutes.

If curing takes place in a convection curing device, the curing time can be measured by any of the methods described above.

In certain embodiments, the curing temperature is defined as a target temperature range, for example, the curing temperature is defined as a target inlet air temperature range or a target exhaust air temperature range. In such embodiments, the starting point of the curing step is defined to be the point in time when the lower limit of the target temperature range is reached, and the end point of the curing step is defined to be the point in time when the heating is stopped or at least reduced, and the temperature subsequently drops below the lower limit of the target temperature range by more than about 10°C and/or below the lower limit of the softening temperature range of polyethylene oxide, for example, below about 62°C.

The curing time, i.e., the time period the formulation is subjected to the curing temperature, which can, for example, be measured according to the methods described above, is at least about 1 minute or at least about 5 minutes. The curing time may vary from about 1 minute to about 24 hours, from about 5 minutes to about 20 hours, from about 10 minutes to about 15 hours, from about 15 minutes to about 10 hours, or from about 30 minutes to about 5 hours depending on the specific formulation and the curing temperature. According to certain embodiments, the curing time varies from about 15 minutes to about 30 minutes. According to further embodiments, wherein the curing temperature is at least about 60°C, at least about 62°C, at least about 68°C, at least about 70°C, at least about 72°C or at least about 75°C, or varies from about 62°C to about 85°C or from about 65°C to about 85°C, then the curing time is preferably at least about 15 minutes, at least about 30 minutes, at least about 60 minutes, at least about 75 minutes, at least about 90 minutes or at least about 120 minutes. In preferred embodiments, wherein the curing temperature is, for example, at least about 62°C, at least about 68°C, at least about 70°C, at least about 72°C or at least about 75°C, or ranges from about 62°C to about 80°C, from about 65°C to about 80°C, from about 68°C to about 80°C, from about 70°C to about 80°C or from about 72°C to about 80°C, then the curing time is preferably at least about 1 minute, at least about 5 minutes, at least about 10 minutes, at least about 15 minutes or at least about 30 minutes. In certain such embodiments, the curing time can be chosen to be as short as possible while still achieving the desired result (e.g., increased tamper resistance). For example, the curing time preferably does not exceed about 5 hours, does not exceed about 3 hours or does not exceed about 2 hours. Preferably, the curing time is in the range of from about 1 minute to about 5 hours, from about 5 minutes to about 3 hours, from about 15 minutes to about 2 hours, or from about 15 minutes to about 1 hour. Any combination of the curing temperatures and the curing times as disclosed herein lies within the scope of the present invention.

In certain embodiments, the composition is only subjected to the curing temperature until the polyethylene oxide present in the formulation has reached its softening temperature and/or at least partially melts. In certain such embodiments, the curing time may be less than about 5 minutes, for example the curing time may vary from greater than 0 minutes to about 3 hours, from about 1 minute to about 2 hours or from about 2 minutes to about 1 hour. Instant curing is possible by choosing a curing device which allows for an instant heating of the polyethylene oxide in the formulation to at least its softening temperature, so that the polyethylene oxide at least partially melts. Such curing devices are, for example, microwave ovens, ultrasound devices, light irradiation apparatus such as UV-irradiation apparatus, ultra-high frequency (UHF) fields or any other apparatus known to the person skilled in the art.

The size of the formulation may determine the required curing time and curing temperature to achieve the desired tamper resistance.

In certain embodiments, the curing step leads to a decrease in the density of the formulation, such that the density of the cured formulation is lower than the density of the formulation prior to the curing step. Preferably, the density of the cured formulation in comparison to the density of the uncured formulation decreases by at least about 0.5%. More preferably, the density of the cured formulation in comparison to the density of the uncured formulation decreases by at least about 0.7%, at least about 0.8%, at least about 1.0%, at least about 2.0% or at least about 2.5%.

In certain embodiments, the solid controlled release dosage form is cured at a temperature of at least the softening point of the polyethylene oxide for at least 1 minute, at least 5 minutes or at least 15 minutes.

In other embodiments, the solid controlled release dosage form is cured at a temperature of at least the softening point of the polyethylene oxide from about 1 minute to about 48 hours, from about 5 minutes to about 24 hours, from about 15 minutes to about 1 hour or about 30 minutes.

The solid controlled release dosage form can be cured, e.g., at a temperature of at least about 60° C, at least about 65° C, at least about 70° C, at least about 75° C or at a temperature of about 72° C.

In alternative embodiments, the solid controlled release dosage form can be cured at a temperature from about 60° C to about 90° C, from about 62° C to about 72° C, from about 65° C to about 85° C, from about 70° C to about 80° C, from about 75° C to about 80° C or from about 70° C to about 75° C.

### FLATTENING PROCEDURES

In certain embodiments, dosage forms of the present invention may be flattened without substantially compromising the release of the active or the integrity of the dosage form. Flatness is described in terms of the thickness of the smallest diameter of the flattened shape compared to the thickness of the smallest diameter of the non-flattened shape. This comparison is expressed in % thickness, based on either (i) the thickness of the smallest diameter of the non-flattened shape when the initial shape is non-spherical or (ii) the thickness of the diameter when the initial shape is spherical. The thickness may be measured using a thickness gauge (e.g., a digital thickness gauge or digital caliper). The flattening force may be applied by any possible method. For purposes of testing the dosage forms of the present invention, a carver style bench press may be used (unless otherwise specified) so as to achieve the target flatness or reduced thickness. According to certain embodiments of the invention, the flattening does not result in breaking of the dosage form into separate pieces; however, edge splits and cracks may occur.

In certain embodiments of the invention, a hammer can be used for flattening a dosage form. In such a process, hammer strikes can be manually applied from a direction substantially normal to the thickest dimension of the dosage form. The flatness is then described in the same manner as disclosed above.

In other embodiments, flattening can be measured relative to breaking strength or hardness tests, as described in Remington's Pharmaceutical Sciences, 18th edition, 1990, Chapter 89 "Oral Solid Dosage Forms", pages 1633-1665, using the Schleuniger Apparatus. In such an embodiment, the dosage form is pressed between a pair of flat plates arranged in parallel such that the force is applied substantially normal to the thickest dimension of the dosage form, thereby flattening the dosage form. The flattening of the dosage form may be described in terms of % flattening, based on the thickness of the dimension being flattened before conducting the breaking strength test. The breaking strength (or hardness) is defined as the force at which the tested dosage form breaks. Dosage forms that do not break, but which are deformed due to a force applied, are considered to be break-resistant at that particular force.

The term "resistant to crushing" is defined for the purposes of certain embodiments of the present invention as referring to dosage forms that can be flattened with a bench press as described above, without breaking, to no more than about 60% thickness, preferably to no more than about 50% thickness, more preferably to no more than about 40% thickness, even more preferably to no more than about 30% thickness, and most preferably to no more than about 20% thickness, 10% thickness or 5% thickness.

In certain embodiments, the amount of active agent (e.g., opioid analgesic) released at 0.5 hour from a flattened dosage form deviates no more than about 10 % points, 15 % points or 20% points from the amount released at 0.5 hour from a non-flattened dosage form as measured by an in-vitro dissolution in a USP Apparatus 1 (basket) at 100 rpm in 900 ml simulated gastric fluid without enzymes (SGF) at 37° C.

In alternative embodiments, the solid controlled release dosage form can be flattened without breaking, wherein the thickness of the dosage form after flattening corresponds to no more than about 60% of the thickness of the dosage form before flattening, or to no more than about 50% of the thickness of the dosage form before flattening, or to no more than about 40% of the thickness of the dosage form before flattening, or to no more than about 30% of the thickness of the dosage form before flattening, or to no more than about 20% of the thickness of the dosage form before flattening.

### USES

The present invention is further directed to the use of a purified neutral acrylic polymer in the preparation of an oral solid dosage form, preferably as described herein.

The present invention is also directed to the use of a blend comprising a purified neutral acrylic polymer and an active agent and optionally polyethylene oxide, in the preparation of an oral solid dosage form, preferably as described herein.

In certain embodiments the blend is in the form of a bulk powder. In certain embodiments the blend comprises less than 20% (w/w) water or less than 10% (w/w) water, and preferably comprises less than 5 % (w/w) water or less than 3% (w/w) water. The blend may further comprise less than 10% (w/w) organic solvents, preferably less than 5 % (w/w) organic solvents, most preferably less than 3% (w/w) or less than 1% (w/w) organic solvents.

The present invention is also directed to the use of a composition comprising at least one neutral acrylic polymer, at least one active agent, from 0-8% (w/w) of water, and from 0-5 % (w/w) of organic solvents (e.g. methanol or ethanol), in the preparation of an oral solid dosage form. Preferably the composition further comprises a polyethylene oxide.

The present invention is also directed to the use of a solid composition comprising at least one neutral acrylic polymer, and at least one active agent, for the preparation of a solid oral pharmaceutical dosage form. Preferably the composition further comprises a polyethylene oxide.

The present invention is also directed to the use of an oral solid dosage form as described herein in the manufacture of a medicament for the treatment or prevention of a disease. The present invention is also directed to the use of an oral solid dosage form as described herein in the manufacture of a medicament for the treatment of pain, wherein the oral solid dosage form comprises an opioid agonist.

The present invention is also directed to an oral solid dosage form as described herein for use in the treatment of pain, wherein the oral solid dosage form comprises an opioid agonist.

### FURTHER EMBODIMENTS

The present invention is also directed to the following further embodiments of items 1 to 102:
1. An oral solid dosage form comprising purified neutral acrylic polymer and a prophylactically or therapeutically effective amount of an active agent.
2. The oral solid dosage form of item 1, wherein the purified neutral acrylic polymer is derived from a dried neutral acrylic polymer aqueous dispersion.
3. The oral solid dosage form of item 1, wherein the purified neutral acrylic polymer is derived from a vacuum dried neutral acrylic polymer aqueous dispersion.
4. The oral solid dosage form of item 1, wherein the purified neutral acrylic polymer is derived from a lyophilized neutral acrylic polymer aqueous dispersion.
5. The oral solid dosage form of item 1, wherein the purified neutral acrylic polymer is derived from a pan dried neutral acrylic polymer aqueous dispersion.
6. The oral solid dosage form of item 1, wherein the purified neutral acrylic polymer is derived from an oven dried neutral acrylic polymer aqueous dispersion.
7. The oral solid dosage form of any of items 2-6, wherein the purified neutral acrylic polymer is milled.
8. The oral solid dosage form of item 1, wherein the purified neutral acrylic polymer is derived from an aqueous dispersion comprising from about 20% (w/w) to about 50% (w/w) neutral acrylic polymer.
9. The oral solid dosage form of item 1, wherein the purified neutral acrylic polymer is derived from an aqueous suspension comprising from about 30%(w/w) to about 40%(w/w) neutral acrylic polymer.
10. The oral solid dosage form of item 1, comprising an effective amount of the purified neutral acrylic polymer to provide a controlled release of the active agent.
11. The oral solid dosage form of item 1, comprising from about 10% (w/w) to about 90% (w/w) purified neutral acrylic polymer.
12. The oral solid dosage form of item 1, comprising from about 20% (w/w) to about 80% (w/w) purified neutral acrylic polymer.
13. The oral solid dosage form of item 1, comprising from about 30% (w/w) to about 70% (w/w) purified neutral acrylic polymer.
14. The oral solid dosage form of item 1, comprising from about 40% (w/w) to about 60% (w/w) purified neutral acrylic polymer.
15. The oral solid dosage form of item 1, wherein the purified neutral acrylic polymer comprises less than about 5% (w/w) water.
16. The oral solid dosage form of item 1, wherein the purified neutral acrylic polymer comprises less than about 3% (w/w) water.
17. The oral solid dosage form of item 1, wherein the purified neutral acrylic polymer comprises less than about 1% (w/w) water.
18. The oral solid dosage form of item 1, comprising from about 1% (w/w) to about 50% (w/w) active agent.
19. The oral solid dosage form of item 1, comprising from about 5% (w/w) to about 40% (w/w) active agent.
20. The oral solid dosage form of item 1, comprising from about 10% (w/w) to about 30% (w/w) active agent.
21. The oral solid dosage form of item 1, comprising from about 15% (w/w) to about 25% (w/w) active agent.
22. The oral solid dosage form of item 1, further comprising at least one excipient selected from the group consisting of polymers, poloxamers, bulking agents, release modifying agents, plasticizers, stabilizers, diluents, lubricants, binders, granulating aids, colorants, flavorants, and glidants.
23. The oral solid dosage form of item 22, wherein the excipient is a polymer.
24. The oral solid dosage form of item 23, wherein the polymer is polyethylene oxide.
25. The oral solid dosage form of item 24, wherein the polyethylene oxide is a low molecular weight polyethylene oxide.
26. The oral solid dosage form of item 25, wherein the polyethylene oxide has an average molecular weight from about 10,000 Daltons to about 750,000 Daltons.
27. The oral solid dosage form of item 25, wherein the polyethylene oxide has an average molecular weight from about 50,000 Daltons to about 500,000 Daltons.
28. The oral solid dosage form of item 25, wherein the polyethylene oxide has an average molecular weight from about 75,000 Daltons to about 300,000 Daltons.
29. The oral solid dosage form of item 24, comprising from about 5% (w/w) to about 60% (w/w) polyethylene oxide.
30. The oral solid dosage form of item 24, comprising from about 10% (w/w) to about 50% (w/w) polyethylene oxide.
31. The oral solid dosage form of item 24, comprising from about 15% (w/w) to about 40% (w/w) polyethylene oxide.
32. The oral solid dosage form of item 24, comprising from about 20% (w/w) to about 30% (w/w) polyethylene oxide.
33. The oral solid dosage form item 24, comprising from about 10%(w/w) to about 90%(w/w) purified neutral acrylic polymer, from about 1%(w/w) to about 50%(w/w) active agent and from about 5%(w/w) to about 60%(w/w) polyethylene oxide.
34. The oral solid dosage form item 24, comprising from about 20%(w/w) to about 80%(w/w) purified neutral acrylic polymer, from about 5%(w/w) to about 40%(w/w) active agent and from about 10%(w/w) to about 50%(w/w) polyethylene oxide.
35. The oral solid dosage form item 24, comprising from about 30%(w/w) to about 70%(w/w) purified neutral acrylic polymer, from about 10%(w/w) to about 30%(w/w) active agent and from about 15%(w/w) to about 40%(w/w) polyethylene oxide.
36. The oral solid dosage form of item 24, comprising from about 40%(w/w) to about 60%(w/w) purified neutral acrylic polymer, from about 15%(w/w) to about 25%(w/w) active agent and from about 20%(w/w) to about 30%(w/w) polyethylene oxide.
37. The oral solid dosage form of item 1, wherein the purified neutral acrylic polymer and the active agent are in the form of an extruded blend.
38. The oral solid dosage form of item 24, wherein the purified neutral acrylic polymer, the active agent and the polyethylene oxide are in the form of an extruded blend.
39. The oral solid dosage form of item 37 or 38, wherein the extruded blend is in the form of a unitary dosage form.
40. The oral solid dosage form of item 37 or 38, wherein the extruded blend is in the form of multiparticulates.
41. The oral solid dosage form of item 40, wherein the multiparticulates are in the form of pellets.
42. The oral solid dosage form of item 41, wherein the pellets have a mean diameter from about 0.1 mm to about 5 mm and a mean height from about 0.1 mm to about 5 mm.
43. The oral solid dosage form of item 41, wherein the pellets have a mean diameter from about 0.5 mm to about 4 mm and a mean height from about 0.5 mm to about 4 mm.
44. The oral solid dosage form of item 41, wherein the pellets have a mean diameter from about 1 mm to about 3 mm and a mean height from about 0.5 mm to about 4 mm.
45. The oral solid dosage form of item 41, wherein the pellets have a mean diameter from about 1.5 mm to about 2.5 mm and a mean height from about 1.5 mm to about 2.5 mm.
46. The oral solid dosage form of item 40, wherein the particles are in the form of spheres.
47. The oral solid dosage form of item 36, wherein the spheres have a mean diameter from about 0.1 mm to about 5 mm.
48. The oral solid dosage form of item 36, wherein the spheres have a mean diameter from about 0.5 mm to about 4 mm.
49. The oral solid dosage form of item 36, wherein the spheres have a mean diameter from about 1 mm to about 3 mm.
50. The oral solid dosage form of item 36, wherein the spheres have a mean diameter from about 1.5 mm to about 2.5 mm.
51. The oral solid dosage form of item 40, wherein the multiparticulates are contained in a pharmaceutically acceptable capsule.
52. The oral solid dosage form of item 40, wherein the multiparticulates are compressed.
53. The oral solid dosage form of item 1 or 24, wherein the active agent is selected from the group consisting of ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha-two-adrenergic agonists, analgesics, antipyretics, anti-inflammatory agents, androgens, local and general anesthetics, antiaddictive agents, antiandrogens, antiarrhythmic agents, antiasthmatic agents, anticholinergic agents, anticholinesterase agents, anticoagulants, antidiabetic agents, antidiarrheal agents, antidiuretic, antiemetic and prokinetic agents, antiepileptic agents, antiestrogens, antifingal agents, antihypertensive agents, antimicrobial agents, antimigraine agents, antimuscarinic agents, antineoplastic agents, antiparasitic agents, antiparkinson's agents, antiplatelet agents, antiprogestins, antischizophrenia agents, antithyroid agents, antitussives, antiviral agents, atypical antidepressants, azaspirodecanediones, barbituates, benzodiazepines, benzothiadiazides, beta-adrenergic agonists, beta-adrenergic antagonists, selective beta-one-adrenergic antagonists, selective beta-two-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, contraceptive agents, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, hematopoietic agents, histamines, histamine antagonists, hormones, 5-hydroxytryptamine antagonists, drugs for the treatment of hyperlipoproteinemia, hypnotics, sedatives, immunosupressive agents, laxatives, methylxanthines, moncamine oxidase inhibitors, neuromuscular blocking agents, organic nitrates, opioid agonists, opioid antagonists, pancreatic enzymes, phenothiazines, progestins, prostaglandins, agents for the treatment of psychiatric disorders, retinoids, sodium channel blockers, agents for spasticity and acute muscle spasms, succinimides, testosterones, thioxanthines, thrombolytic agents, thyroid agents, tricyclic antidepressants, inhibitors of tubular transport of organic compounds, drugs affecting uterine motility, vasodilators, vitamins, and mixtures thereof.
54. The oral solid dosage form of item 1 or 24, wherein the active agent is an opioid agonist.
55. The oral solid dosage form of item 54, wherein the opioid agonist is selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof.
56. The oral solid dosage form of item 54, wherein the opioid agonist is selected from the group consisting of codeine, fentanyl, hydromorphone, hydrocodone, oxycodone, dihydrocodeine, dihydromorphine, morphine, tramadol, oxymorphone, pharmaceutically acceptable salts thereof, and mixture thereof.
57. The oral solid dosage form of item 54, wherein the opioid agonist is oxycodone or a pharmaceutically acceptable salt thereof.
58. The oral solid dosage form of item 1 or 24, wherein the active agent is an opioid antagonist.
59. The oral solid dosage form of item 58, wherein the opioid antagonist is selected from the group consisting of amiphenazole, naltrexone, methylnaltrexone, naloxone, nalbuphine, nalorphine, nalorphine dinicotinate, nalmefene, nadide, levallorphan, cyclozocine, pharmaceutically acceptable salts thereof and mixtures thereof.
60. The oral solid dosage form of item 1 or 24, wherein the active agent is a non-opioid analgesic.
61. The oral solid dosage form of item 60, wherein the non-opioid analgesic is a non-steroidal anti-inflammatory agent selected from the group consisting of aspirin, celecoxib, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, pharmaceutically acceptable salts thereof and mixtures thereof.
62. The oral solid dosage form of item 54, wherein the amount of opioid agonist thereof released at 1 hour in 900 mL 0.1 N HCl (pH 1.5) with 40% EtOH using USP Apparatus II at 50 rpm is not more than the amount of opioid agonist released at 1 hour in 900 mL 0.1 N HCl (pH 1.5) using USP Apparatus II at 50 rpm.
63. The oral solid dosage form of item 54, wherein the amount of opioid agonist thereof released at 1 hour in 900 mL 0.1 N HCl (pH 1.5) with 40% EtOH using USP Apparatus II at 50 rpm is less than the amount of opioid agonist released at 1 hour in 900 mL 0.1 N HCl (pH 1.5) using USP Apparatus II at 50 rpm.
64. The oral solid dosage form of item 54, wherein the amount of opioid agonist thereof released at 1 hour in 900 mL 0.1 N HCl (pH 1.5) with 40% EtOH using USP Apparatus II at 50 rpm is within 25%(w/w) of the amount of opioid agonist released at 1 hour in 900 mL 0.1 N HCl (pH 1.5) using USP Apparatus II at 50 rpm.
65. The oral solid dosage form of item 54, wherein the amount of opioid agonist thereof released at 1 hour in 900 mL 0.1 N HCl (pH 1.5) with 40% EtOH using USP Apparatus II at 50 rpm is within 10%(w/w) of the amount of opioid agonist released at 1 hour in 900 mL 0.1 N HCl (pH 1.5) using USP Apparatus II at 50 rpm.
66. The oral solid dosage form of item 54, wherein the amount of opioid agonist thereof released at 2 hour in 900 mL 0.1 N HCl (pH 1.5) with 40% EtOH using USP Apparatus II at 50 rpm is not more than the amount of opioid agonist released at 2 hour in 900 mL 0.1 N HCl (pH 1.5) using USP Apparatus II at 50 rpm.
67. The oral solid dosage form of item 54, wherein the amount of opioid agonist thereof released at 2 hour in 900 mL 0.1 N HCl (pH 1.5) with 40% EtOH using USP Apparatus II at 50 rpm is less than the amount of opioid agonist released at 2 hour in 900 mL 0.1 N HCl (pH 1.5) using USP Apparatus II at 50 rpm.
68. The oral solid dosage form of item 54, wherein the amount of opioid agonist thereof released at 2 hour in 900 mL 0.1 N HCl (pH 1.5) with 40% EtOH using USP Apparatus II at 50 rpm is within 25%(w/w) of the amount of opioid agonist released at 2 hour in 900 mL 0.1 N HCl (pH 1.5) using USP Apparatus II at 50 rpm.
69. The oral solid dosage form of item 54, wherein the amount of opioid agonist thereof released at 2 hour in 900 mL 0.1 N HCl (pH 1.5) with 40% EtOH using USP Apparatus II at 50 rpm is within 10%(w/w) of the amount of opioid agonist released at 2 hour in 900 mL 0.1 N HCl (pH 1.5) using USP Apparatus II at 50 rpm.
70. The oral solid dosage form of item 54, wherein the amount of opioid agonist thereof released at 4 hour in 900 mL 0.1 N HCl (pH 1.5) with 40% EtOH using USP Apparatus II at 50 rpm is not more than the amount of opioid agonist released at 4 hour in 900 mL 0.1 N HCl (pH 1.5) using USP Apparatus II at 50 rpm.
71. The oral solid dosage form of item 54, wherein the amount of opioid agonist thereof released at 4 hour in 900 mL 0.1 N HCl (pH 1.5) with 40% EtOH using USP Apparatus II at 50 rpm is less than the amount of opioid agonist released at 4 hour in 900 mL 0.1 N HCl (pH 1.5) using USP Apparatus II at 50 rpm.
72. The oral solid dosage form of item 54, wherein the amount of opioid agonist thereof released at 4 hour in 900 mL 0.1 N HCl (pH 1.5) with 40% EtOH using USP Apparatus II at 50 rpm is within 25%(w/w) of the amount of opioid agonist released at 4 hour in 900 mL 0.1 N HCl (pH 1.5) using USP Apparatus II at 50 rpm.
73. The oral solid dosage form of item 54, wherein the amount of opioid agonist thereof released at 4 hour in 900 mL 0.1 N HCl (pH 1.5) with 40% EtOH using USP Apparatus II at 50 rpm is within 10%(w/w) of the amount of opioid agonist released at 4 hour in 900 mL 0.1 N HCl (pH 1.5) using USP Apparatus II at 50 rpm.
74. The oral solid dosage form of item 54, wherein the amount of opioid agonist released at 1 hour is from about 10%(w/w) to about 30%(w/w) as measured by an in-vitro dissolution in a USP Apparatus 1 (basket) at 100 rpm in 900 ml simulated gastric fluid without enzymes (SGF) at 37° C.
75. The oral solid dosage form of item 54, wherein the amount of opioid agonist released at 2 hours is from about 25%(w/w) to about 50%(w/w) as measured by an in-vitro dissolution in a USP Apparatus 1 (basket) at 100 rpm in 900 ml simulated gastric fluid without enzymes (SGF) at 37° C.
76. The oral solid dosage form of item 54, wherein the amount of opioid agonist released at 4 hours is from about 40%(w/w) to about 80%(w/w) as measured by an in-vitro dissolution in a USP Apparatus 1 (basket) at 100 rpm in 900 ml simulated gastric fluid without enzymes (SGF) at 37° C.
77. The oral solid dosage form of item 54, wherein the amount of opioid agonist released at 8 hours is from about 65%(w/w) to about 95%(w/w) as measured by an in-vitro dissolution in a USP Apparatus 1 (basket) at 100 rpm in 900 ml simulated gastric fluid without enzymes (SGF) at 37° C.
78. The oral solid dosage form of item 54, wherein the amount of opioid agonist released at 12 hours is greater than about 80%(w/w) as measured by an in-vitro dissolution in a USP Apparatus 1 (basket) at 100 rpm in 900 ml simulated gastric fluid without enzymes (SGF) at 37° C.
79. The oral solid dosage form of item 54, wherein the amount of opioid agonist released at 1 hour from about 15%(w/w) to about 25%(w/w) as measured by an in-vitro dissolution in a USP Apparatus 1 (basket) at 100 rpm in 900 ml simulated gastric fluid without enzymes (SGF) at 37° C.
80. The oral solid dosage form of item 54, wherein the amount of opioid agonist released at 2 hours is from about 30%(w/w) to about 40%(w/w) as measured by an in-vitro dissolution in a USP Apparatus 1 (basket) at 100 rpm in 900 ml simulated gastric fluid without enzymes (SGF) at 37° C.
81. The oral solid dosage form of item 54, wherein the amount of opioid agonist released at 4 hours is from about 55%(w/w) to about 75%(w/w) as measured by an in-vitro dissolution in a USP Apparatus 1 (basket) at 100 rpm in 900 ml simulated gastric fluid without enzymes (SGF) at 37° C.
82. The oral solid dosage form of item 54, wherein the amount of opioid agonist released at 8 hours is from about 75%(w/w) to about 85%(w/w) as measured by an in-vitro dissolution in a USP Apparatus 1 (basket) at 100 rpm in 900 ml simulated gastric fluid without enzymes (SGF) at 37° C.
83. The oral solid dosage form of item 54, wherein the amount of opioid agonist released at 12 hours is greater than about 90%(w/w) as measured by an in-vitro dissolution in a USP Apparatus 1 (basket) at 100 rpm in 900 ml simulated gastric fluid without enzymes (SGF) at 37° C.
84. The oral solid dosage form of item 54, wherein the amount of opioid agonist released at 1 hour is from about 10%(w/w) to about 30%(w/w); the amount of opioid agonist released at 2 hours is from about 25%(w/w) to about 50%(w/w); the amount of opioid agonist released at 4 hours is from about 40%(w/w) to about 80%(w/w); the amount of opioid agonist released at 8 hours is from about 65%(w/w) to about 95%(w/w) and the amount of opioid agonist released at 12 hours is greater than about 80%(w/w); as measured by an in-vitro dissolution in a USP Apparatus 1 (basket) at 100 rpm in 900 ml simulated gastric fluid without enzymes (SGF) at 37° C.
85. The oral solid dosage form of item 54, wherein the amount of opioid agonist released at 1 hour is from about 15%(w/w) to about 25%(w/w); the amount of opioid agonist released at 2 hours is from about 30%(w/w) to about 40%(w/w); the amount of opioid agonist released at 4 hours is from about 55%(w/w) to about 75%(w/w); the amount of opioid agonist released at 8 hours is from about 75%(w/w) to about 85%(w/w) and the amount of opioid agonist released at 12 hours is greater than about 90%(w/w); as measured by an in-vitro dissolution in a USP Apparatus 1 (basket) at 100 rpm in 900 ml simulated gastric fluid without enzymes (SGF) at 37° C.
86. The oral solid dosage form of item 54, wherein the dosage form can be flattened without breaking, wherein the thickness of the dosage form after flattening corresponds to no more than about 60% of the thickness of the dosage form before flattening.
87. The oral solid dosage form of item 54, wherein the dosage form can be flattened without breaking, wherein the thickness of the dosage form after flattening corresponds to no more than about 50% of the thickness of the dosage form before flattening.
88. The oral solid dosage form of item 54, wherein the dosage form can be flattened without breaking, wherein the thickness of the dosage form after flattening corresponds to no more than about 40% of the thickness of the dosage form before flattening.
89. The oral solid dosage form of item 54, wherein the dosage form can be flattened without breaking, wherein the thickness of the dosage form after flattening corresponds to no more than about 30% of the thickness of the dosage form before flattening.
90. The oral solid dosage form of item 54, wherein the dosage form can be flattened without breaking, wherein the thickness of the dosage form after flattening corresponds to no more than about 20% of the thickness of the dosage form before flattening.
91. The oral solid dosage form of item 54, wherein the amount of opioid agonist released at 0.5 hour from a flattened dosage form deviates no more than about 20% points from a non-flattened dosage form as measured by an in-vitro dissolution in a USP Apparatus 1 (basket) at 100 rpm in 900 ml simulated gastric fluid without enzymes (SGF) at 37° C.
92. The oral solid dosage form of item 54, wherein the amount of opioid agonist released at 0.5 hour from a flattened dosage form deviates no more than about 15% points from a non-flattened dosage form as measured by an in-vitro dissolution in a USP Apparatus 1 (basket) at 100 rpm in 900 ml simulated gastric fluid without enzymes (SGF) at 37° C.
93. The oral solid dosage form of item 54, wherein the amount of opioid agonist released at 0.5 hour from a flattened dosage form deviates no more than about 10% points from a non-flattened dosage form as measured by an in-vitro dissolution in a USP Apparatus 1 (basket) at 100 rpm in 900 ml simulated gastric fluid without enzymes (SGF) at 37° C.
94. A method of treating a disease or condition comprising administering an oral solid dosage form of any of items 1-93 to a patient in need thereof.
90. A method of treating pain comprising administering an oral solid dosage form of any of items 54-57 and 60-93 to a patient in need thereof.
95. A method of preparing an oral solid dosage form of any of items 1-23, 37 and 39-93 comprising (i) mixing in an extruder the purified neutral acrylic polymer and the active agent; (ii) extruding the mixture as a strand; (iii) cooling the strand; and (iv) dividing the strand into unit doses.
96. A method of preparing an oral solid dosage form of any of items 24-36 and 38-93 comprising (i) mixing in an extruder the purified neutral acrylic polymer, the active agent; and the polyethylene oxide (ii) extruding the mixture as a strand; (iii) cooling the strand; and (iv) dividing the strand into unit doses.
97. Purified neutral acrylic polymer.
98. Vacuum dried neutral acrylic polymer.
99. Freeze dried neutral acrylic polymer.
100. A bulk powder comprising (i) purified neutral acrylic polymer and (ii) an active agent.
101. A bulk powder comprising (i) vacuum dried neutral acrylic polymer and (ii) an active agent.
102. A bulk powder comprising (i) freeze dried neutral acrylic polymer and (ii) an active agent.

The following examples are set forth to assist in understanding the invention and should not be construed as specifically limiting the invention described and claimed herein. Such variations of the invention, including the substitution of all equivalents now known or later developed, which would be within the purview of those skilled in the art, and changes in formulation or minor changes in experimental design, are to be considered to fall within the scope of the invention incorporated herein.

### EXAMPLES

### Example 1

The formulations of Example 1 were prepared in accordance with the following ingredients of Table 1:

**TABLE 1**

| | Amt/unit (%) | | Amt/unit (mg) | | Amt/Batch (gm) | |
|---|---|---|---|---|---|---|
| Ingredient | Sub-Lot A | Sub-Lot B | Sub-Lot A | Sub-Lot B | Sub-Lot A | Sub-Lot B |
| Oxycodone HCI | 20% | 20% | 30 | 30 | 100 | 100 |
| Eudragit NE 40 D Solids Vacuum Dried | 60% | 60% | 90 | 90 | 300 | 300 |
| PEO N10 | 20% | -- | 30 | -- | 100 | -- |
| Lutrol Micro 127 MP | -- | 20% | -- | 30 | -- | 100 |
| Total | 100% | 100% | 150 | 150 | 500 | 500 |

The formulations were prepared according to the following procedures:

### 1. Drying and Milling.

Approximately 700 grams of Eudragit NE 40D Solids was prepared by drying approximately 1,750 grams of aqueous Eudragit NE 40D dispersion in a vacuum oven to yield sheets of polymer. The sheets of polymer were sliced with a paper cutter into 2.5 inch squares. The squares were then milled in a Waring blender with dry ice. Then, 600 grams of the milled polymer was passed through a #14 mesh screen.

### 2. Blending.

Sub-Lot A. The above-indicated amounts of Eudragit NE 40D and PEO N10 were weighed into a tared 16-ounce jar. The jar was rotated until the materials were sufficiently blended. Then, 100 grams of oxycodone HCl was added to the jar, and the jar was further rotated until a uniform blend was achieved.

Sub-Lot B. The above-indicated amounts of Eudragit NE 40D and Lutrol were weighed into a tared plastic bag and blended for about 20 seconds. Then, 100 grams of oxycodone HCl was added and the mixture was blended for an additional 30 seconds.

### 3. Extrusion. The blends from above were extruded using a ZSE Extruder according to the following parameters in Table 1A:

**TABLE 1A**

| Time | Sub-Lot B, Run 1 | Sub-Lot B, Run 2 | Sub-Lot B, Run 3 | Sub-Lot A, Run 1 | Sub-Lot A, Run 2 | Sub-Lot A, Run 3 |
|---|---|---|---|---|---|---|
| Screw Speed (rpm) | 50 | 50 | 50 | 50 | 50 | 50 |
| Motor Torque (%) | 24% | 24% | 27% | 53% | 60% | 55% |
| Melt Pressure (psi) | 370 | 640 | 1060 | 630 | 2520 | 1560 |
| Melt Temp (°C) | 108 | 111 | 111 | 103 | 103 | 101 |
| Vacuum (m bar) | --- | 953 | 953 | 946 | 946 | 944 |
| Feed Rate (g/min) | 24 | 24 | 24 | 24 | 24 | 24 |
| **Temp (°C)** | | | | | | |
| Zone 1 | 12.4 | 12.4 | 12.4 | 12.3 | 13.1 | 13.8 |
| Zone 2 | 40 | 40 | 40 | 40 | 40 | 40 |
| Zone 3 | 75 | 75 | 75 | 75 | 75 | 75 |
| Zone 4 | 100 | 100 | 100 | 100 | 100 | 100 |
| Zone 5 | 100 | 100 | 100 | 100 | 100 | 100 |
| Zone 6 | 100 | 100 | 100 | 100 | 100 | 100 |
| Zone 7 | 100 | 100 | 100 | 100 | 100 | 100 |
| Zone 8 | 100 | 100 | 100 | 100 | 100 | 100 |
| Zone 9 | 100 | 100 | 100 | 100 | 100 | 100 |
| Zone 10 | 100 | 100 | 100 | 100 | 100 | 100 |
| Zone 11 Main Gate Adapter (MGA) | 100 | 103 | 104.7 | 103.3 | 102.7 | 99.5 |
| Zone 12 Die | 100 | 91.9 | 111..5 | 100.8 | 93.9 | 100.6 |
| **Strand Thickness (mm) and Die type** | No Die .5 inch rod collected | Vary 22 holes x 2 mm | Vary 20 holes x 1mm | No Die .5 inch strand collected | Vary 20 holes x 1mm | Vary 22 holes x 2mm |

The bulk extrudates from the various runs comprised .5 inch strands, 3mm strands, and 1mm strands.

### 4. Tablet/pellet preparation. Individual doses were created as follows:

Sub-Lot A. The following tablets/pellets were made using the Sub-Lot A blend/extrudate:
- Four .25 inch tablets were made by compressing the pre-extrusion blend. Of these, two tablets were cured in the loss on drying (LOD) tester for 30 minutes at 105°C. The weight of the tablets ranged from 105.6 mg to 113.5 mg, and the thickness ranged from 3.83 mm to 4.30 mm.
- .25 inch tablets were punched out from a slice cut from a .5 inch strands from sub-lot A, run 1 using an F3 press under high pressure. The weight of the tablets ranged from 74.1 mg to 105.3 mg, and the thickness ranged from 2.30 mm to 3.24 mm.
- 2mm x 2mm pellets were cut from sections of the 1 mm bulk strands from sub-lot A, run 2 that had a diameter of about 2mm (due to die swell).

Sub-Lot B. The following tablets/pellets were made using the Sub-Lot B extrudate:
- .25 inch tablets were punched out from a slice cut from a .5 inch strands from sub-lot B, run 1 using an F3 press under high pressure. The weight of the tablets ranged from 86.8 mg to 112.8 mg, and the thickness ranged from 2.72 mm to 3.65 mm.
- 13/32 inch (0.4063 inch) tablets were punched out from a slice cut from a .5 inch strands from sub-lot B, run 1. The weight of the tablets ranged from 324.7 mg to 536.7 mg, and the thickness ranged from 3.75 mm to 6.67 mm.
- 2mm x 2mm pellets were cut from sections of the 1mm bulk extrudate from sub-lot B, run 3 where the diameter had expanded to about 2mm (due to die swell).

### 5. Dissolution Testing. Tablets and pellets from Sub-Lot A and Sub-Lot B were subjected to dissolution testing and the results are set forth in Table 1B

The dissolution parameters were as follows:
Media: SGF pH actual of 1.15 (target 1.2). Baskets @ 100 RPM in 900 ml of SGF. The system was a UV flow through. Data was normalized by setting the signal for 2mm x 2mm pellets from sample B, run 3, at 360 min to 100% released. The other curves were adjusted by sample weight relative to the sample weight of 2mm x 2mm pellets from sublot B, run 3, corresponding to 40 mg active. These all had the same active agent concentration of about 20%.

**TABLE 1B**

| **Time (min)** | **Sub-Lot A .25 inch tab 20 mg (from rod)** | **Sub-Lot A .25 inch tab 20 mg (from rod)** | **Sub-Lot A 2mm x 2mm pellets 40 mg** | **Sub-Lot A 2mm x 2mm pellets 40 mg** | **Sub-Lot B .25 inch tab 20 mg** | **Sub-Lot B 13/32 inch tab 90 mg** | **Sub-Lot B 13/32 inch tab 90 mg** | **Sub-Lot B 2mm x 2mm pellets 40 mg** |
|---|---|---|---|---|---|---|---|---|
| 0 | 0.0% | 0.0% | 0.0% | 0.0% | 0.0% | 0.0% | 0.0% | 0.0% |
| 30 | 12.1% | 11.9% | 10.7% | 11.2% | 26.5% | 14.9% | 14.0% | 61.3% |
| 60 | 16.3% | 15.7% | 14.7% | 15.2% | 36.1% | 20.9% | 20.2% | 81.0% |
| 120 | 21.6% | 20.6% | 19.6% | 20.1% | 47.5% | 29.0% | 28.0% | 96.3% |
| 180 | 25.1% | 23.9% | 22.9% | 23.3% | 55.0% | 34.5% | 33.3% | 99.5% |
| 240 | 27.8% | 26.3% | 25.3% | 25.7% | 60.4% | 38.4% | 37.5% | 100.0% |
| 480 | 34.4% | 32.3% | 31.4% | 31.5% | 74.1% | 48.8% | 48.1% | 100.0% |
| 720 | 38.1% | 35.8% | 35.1% | 35.0% | 82.0% | 55.1% | 54.4% | 100.4% |
| 960 | 40.7% | 38.1% | 37.7% | 37.5% | 85.3% | 59.9% | 58.8% | 100.1% |
| 1200 | 42.4% | 39.8% | 39.6% | 39.4% | 86.6% | 63.5% | 62.4% | 100.0% |
| 1440 | 43.8% | 41.0% | 41.1% | 40.8% | 87.1% | 66.7% | 65.4% | 99.7% |
| 1680 | 43.4% | 41.1% | 41.8% | 41.7% | 86.9% | 69.0% | 67.4% | 99.4% |
| 1920 | 44.4% | 42.1% | 42.9% | 42.7% | 87.0% | 71.7% | 69.8% | 99.7% |
| 2372 | 45.7% | 43.3% | 44.2% | 44.0% | 86.8% | 74.9% | 72.7% | 100.2% |

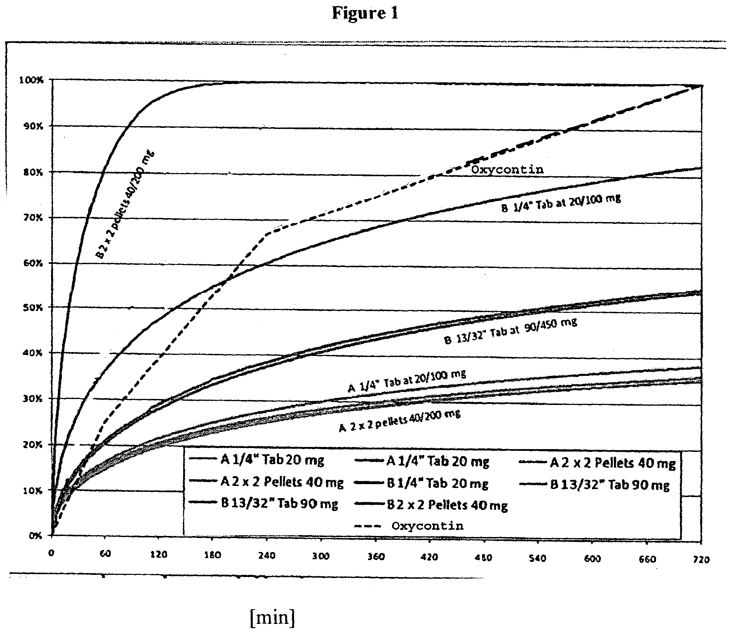

Figure 1 depicts a graphical view of the dissolution data for the sample tablets and pellets of sub-lots A and B. The target identified on the graph is the current reformulated Oxycontin®. The dissolution of the Sub-Lot A preparations may be increased with the inclusion of a screening step after blending.

The dissolution of the 13/32 inch tablet from Sub-Lot B is slower than the dissolution of the .25 inch tablet from Sub-Lot B. This demonstrates that a dissolution can be targeted by selecting and/or adjusting the shape of the tablet or pellet.

### Example 2

The formulations of Example 2 were prepared in accordance with the following ingredients of Table 2:

**TABLE 2**

| Ingredient (by % and grams) | Sub-Lot A | Sub-Lot B | Sub-Lot C | Sub-Lot D | Sub-Lot E | Sub-Lot F |
|---|---|---|---|---|---|---|
| Oxycodone HCl | 10.00% | 10.00% | 20.00% | 20.00% | 16.6667% | 16.6667% |
| | 40.00 g | 40.00 g | 80.00 g | 80.00 g | 66.66668 g | 66.66668 g |
| Eudragit NE Solids | 40.00% | 50.00% | 40.00% | 50.00% | 46.6667% | 36.6667% |
| Oven Dried | 16.00 g | 200.00 g | 160.00 g | 200.00 g | 186.66668 g | 146.66668 g |
| PEO N10 | 50.00% | 40.00% | 40.00% | 30.00% | 36.6667% | 46.6667% |
| | 200.00 g | 160.00 g | 160.00 g | 120.00 g | 146.66668 g | 186.66668 g |
| Total | 100.00% | 100.00% | 100.00% | 100.00% | 100.00% | 100.00% |
| | 400.00 g | 400.00 g | 400.00 g | 400.00 g | 400.00004 g | 400.00004 g |

The formulations were prepared by the following procedures:

### 1. Drying.

Eudragit NE was dried in a hot pack oven at 55°C overnight in a layer about 2 mm thick.

### 2. Milling.

The dried Eudragit NE was sliced into small pieces measuring approximately 3 cm² and milled with dry ice in a Waring blender. Then, the milled Eudragit NE was screened through a #18 U.S. mesh screen.

### 3. Blending.

For each sub-lot, the above-indicated amounts of Eudragit NE and PEO N10 were added to a 32-ounce amber glass bottle and blended by rotating the bottle for 30 seconds. Then, the oxycodone HCl was added to the bottle and the bottle was further rotated for 30 seconds to achieve a uniform blend. Each blend was screened through a #12 mesh screen and then further blended for another 30 seconds prior to extrusion.

### 4. Extrusion.

The blends from above were extruded using a ZSE Extruder according to the following parameters in Table 2A:

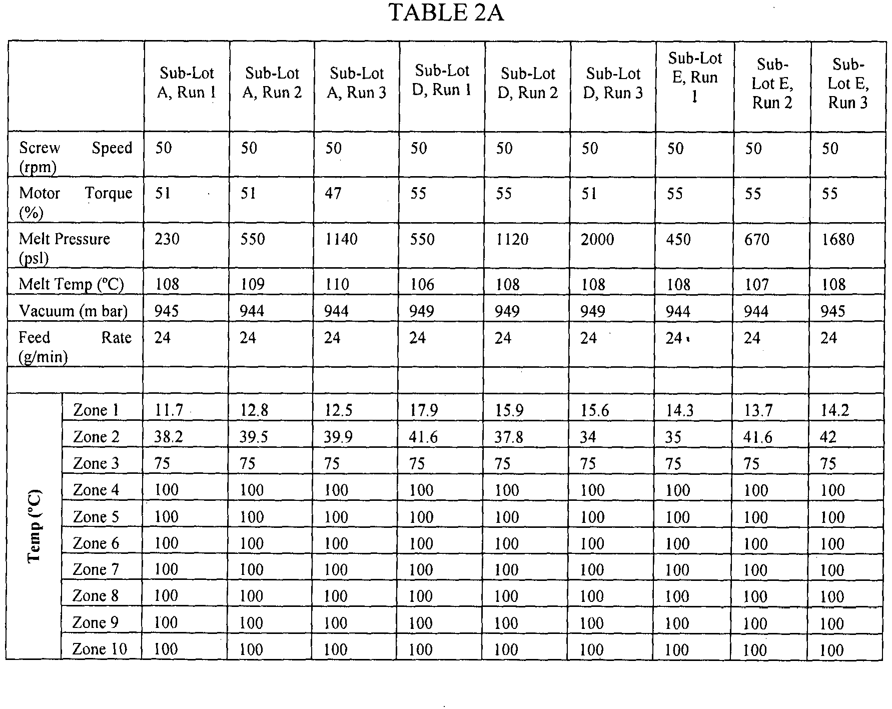

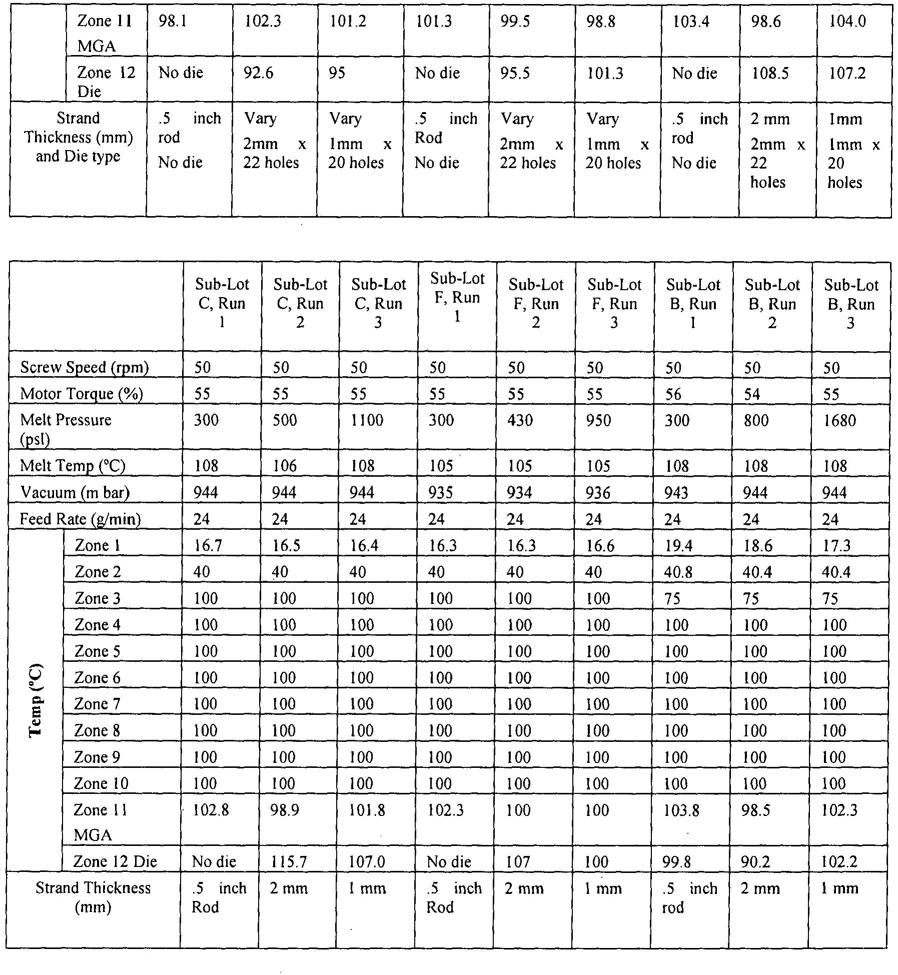

The bulk extrudates from the various runs comprised various thicknesses, including .5 inch rods, 3mm strands, 2mm strands, and 1mm strands.

### 5. Tablet/pellet preparation.

Individual doses were created as follows:

### Sub-Lot B

- .25 inch tablets were cut from the bulk extrudate of sub-lot B, run 1. The tablets had a weight in the range of 161.3 mg to 172.7 mg, and a thickness in the range of 5.31 mm to 5.73 mm.
- 1mm x 1mm pellets, and 2mm x 2mm pellets were also obtained from the strands of bulk extrudate from sub-lot B, runs 2 and 3.

### Sub-Lot C

- The .5 inch rod from sub-lot C, run 1, above was divided into .25 inch tablets. The tablets had a weight in the range of 152.0 mg to 185.8 mg, and a thickness in the range of 4.95 mm to 6.43 mm.
- 1mm x 1mm pellets, and 2mm x 2mm pellets were also obtained from the strands of bulk extrudate from sub-lot C, runs 2 and 3.

### Sub-Lot D

- The .5 inch rod from sub-lot D, run 1 above was divided into .25 inch tablets. The tablets had a weight in the range of 148.9 mg to 178.6 mg, and had a thickness in the range of 4.91 mm to 5.66 mm.
- Pellets measuring approximately 1mm x 1mm and approximately 2mm x 2mm were cut by hand with a razor blade from strands of bulk extrudate from sub-lot D, runs 2 and 3.

### Sub-Lot E

- Pellets measuring approximately 1mm x 1mm and approximately 2mm x 2mm were cut by hand with a razor blade from strands of bulk extrudate from sub-lot E, runs 2 and 3.
- .25 inch tablets were also obtained from the .5 inch rod of bulk extrudate from sub-lot E, run 1.

### Sub-Lot F

- The .5 inch rod from sub-lot F, run 1, was divided into .25 inch tablets. The tablets had a weight in the range of 156.7 mg and 180.2 mg, and a thickness in the range of 5.37 mm and 6.31 mm.
- .25 inch hot molded tablets were also made using the extrudate from sub-lot F, run 1.

### Example 3

The formulations of Example 3 were prepared in accordance with the following ingredients of Table 3:

**TABLE 3**

| Ingredient Wt in (g) and by % | Sub-Lot A | Sub-Lot B | Sub-Lot C | Sub-Lot D |
|---|---|---|---|---|
| Oxycodone HCl | 80.00 g | 60.00 g | 47.10 g | 63.12 g |
| | 20.00% | 15.00% | 12.73% | 15.78% |
| Eudragit NE Solids | 220.00 g | 220.00 g | 222.00 g | 231.52 g |
| Oven Dried | 55.00% | 55.00% | 60.00% | 57.88% |
| PEO N10 | 100.00 g | 120.00 g | 100.90 g | 105.36 g |
| | 25.00% | 30.00% | 27.27% | 26.34% |
| Total | 400.00 g | 400.00 g | 370.00 g | 400.00 g |
| | 100.00% | 100.00% | 100.00% | 100.00% |

The formulations were prepared by the following procedures:

### 1. Drying.

Eudragit NE was dried into thin sheets in a hot pack oven overnight at 55°C.

### 2. Milling.

The dried Eudragit NE was milled with dry ice. The milled Eudragit NE was then passed through a #14 mesh screen.

### 3. Blending.

For each sub-lot, the above-indicated amounts were blended in a jar. The PEO and Eudragit NE were first blended for 20 seconds. Then the oxycodone HCl was added, and the mixture blended for another 20 seconds. The blend was passed through a #8 US mesh screen prior to extrusion.

### 4. Extrusion.

The blends from above were extruded using a ZSE Extruder according to the following parameters in Table 3A:

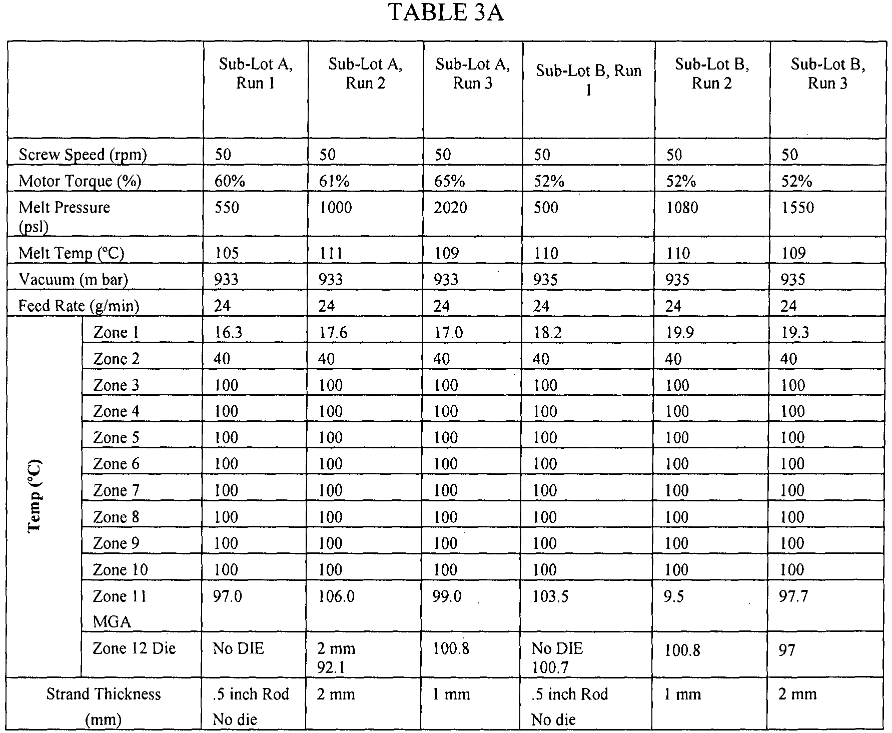

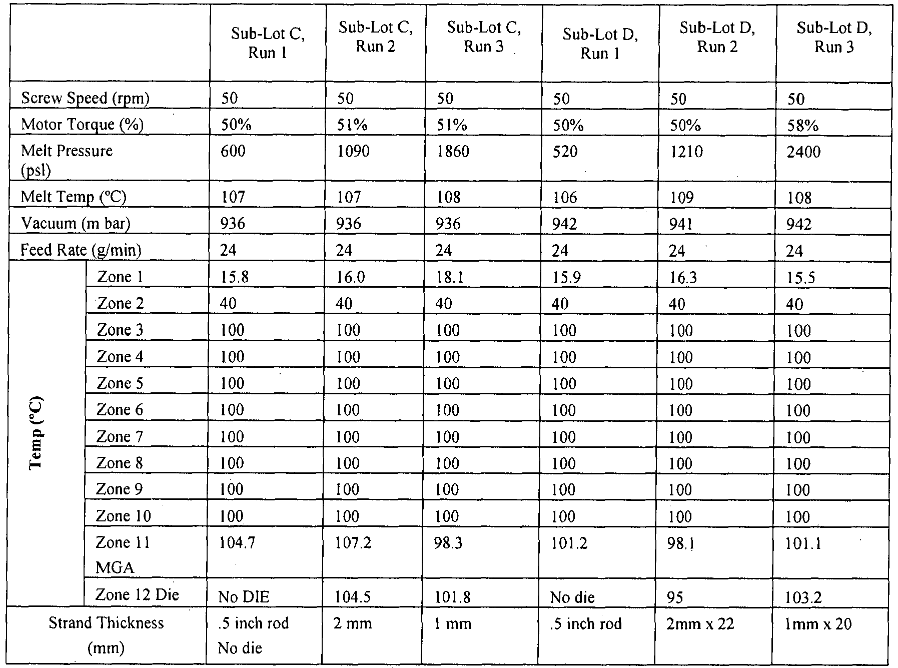

The bulk extrudates from the various runs comprised .5 inch rods, 2mm strands, and 1mm strands.

### 5. Tablet/pellet preparation.

Individual doses were created as follows:

### Sub-Lot A

- Pellets measuring approximately 2mm x 2mm were cut with a razor from the 2mm strand extrudate of sub-lot A, run 2.
- Pellets measuring approximately 1mm x 1mm were cut with a razor from the 1mm strand extrudate of sub-lot A, run 3.

### Sub-Lot B

- Pellets measuring approximately 2mm x 2mm were cut with a razor from the 2mm strand extrudate of sub-lot B, run 3.
- Pellets measuring approximately 1mm x 1mm were cut with a razor from the 1mm strand extrudate of sub-lot B, run 2.

### Sub-Lot C

- Pellets measuring approximately 2mm x 2mm were cut with a razor from the 2mm strand extrudate of sub-lot C, run 2.
- Pellets measuring approximately 1mm x 1mm were cut with a razor from the 1mm strand extrudate of sub-lot C, run 3.

### Sub-Lot D

- Pellets measuring approximately 2mm x 2mm were cut with a razor from the 2mm strand extrudate of sub-lot D, run 2.
- Pellets measuring approximately 1mm x 1mm were cut with a razor from the 1mm strand extrudate of sub-lot D, run 3.
- .25 inch tablets were cut from the .5 inch rod extrudate of sub-lot D, run 1 and hot pressed.

### Example 4

The formulations of Example 4 were prepared in accordance with the following ingredients of Table 4:

**TABLE 4**

| Ingredient (%) and weight (g) | A | B | C |
|---|---|---|---|
| Oxycodone HCI | 20.00% | 15.00% | 15.00% |
| | 80.00 g | 60.00 g | 59.90 g |
| Eudragit NE Solids | 70.00% | 75.00% | 70.00% |
| Oven Dried | 280.00 g | 300.00 g | 280.00 g |
| PEO N10 | 10.00% | 10.00% | 15.00% |
| | 40.00 g | 40.00 g | 60.00 g |
| Total | 100.00% | 100.00% | 100.00% |
| | 400.00 g | 400.00 g | 399.90 g |

The formulations were prepared by the following procedures:

### 1. Drying.

Eudragit NE 40D was dried in an oven (e.g., a Hotpack®) at approx. 50°C until clear (approximately 6 hours). The sheet of dried polymer (approximately 1 mm thick) was cut into squares measuring about .5 inches with a paper cutter.

### 2. Milling.

The squares were milled in a Waring blender with dry ice and passed through a #14 U.S. mesh screen.

### 3. Blending.

For each sub-lot, the above-indicated amounts of milled Eudragit and PEO were added to a 32-ounce wide mouth jar and blended for approximately 1 minute. The oxycodone HCl was added, blended, and discharged through a US mesh screen to remove any lumps that may have formed. The blend was then placed back into the jar and blended for an additional 1 minute.

### 4. Extrusion

The above blends were extruded using a ZSE Extruder according to the following parameters in Table 4A:

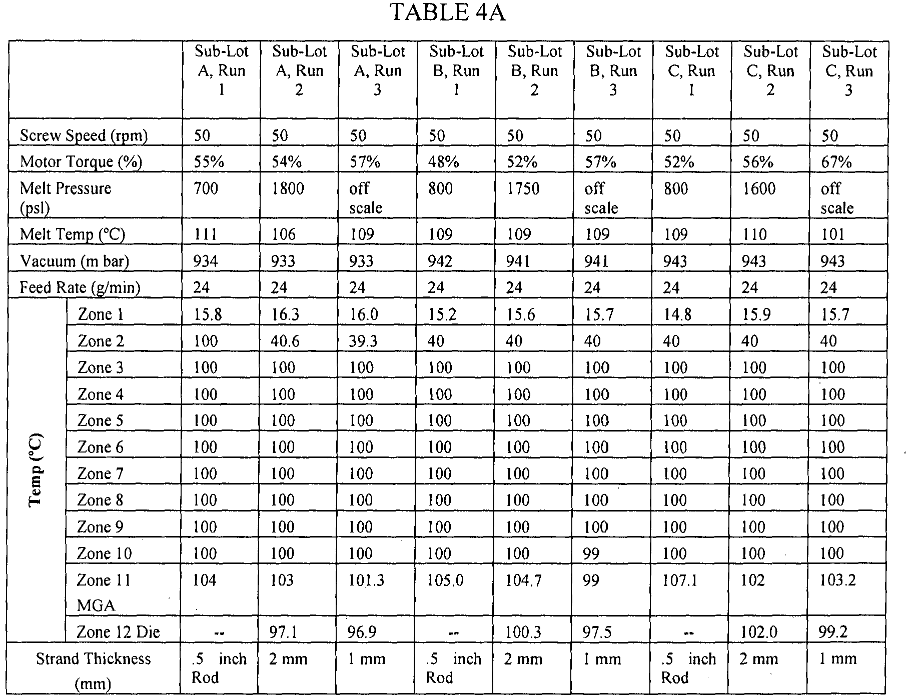

The extrudate included .5 inch rods, 1 mm strands, and 2mm strands.

### 5. Pellet preparation

Individual doses were created as follows:

### Sub-Lot A

- Pellets measuring approximately 1mm x 1mm and 2mm x 2mm were cut by hand with a razor from the extruded strands of sub-lot A, runs 3 and 2.

### Sub-Lot B

- Pellets measuring approximately 1mm x 1mm and 2mm x 2mm were cut by hand with a razor from the extruded strands of sub-lot B, runs 3 and 2.

### Sub-Lot C

- Pellets measuring approximately 1mm x 1mm and 2mm x 2mm were cut by hand with a razor from the extruded strands of sub-lot B, runs 3 and 2.

### 6. Pellet Milling.

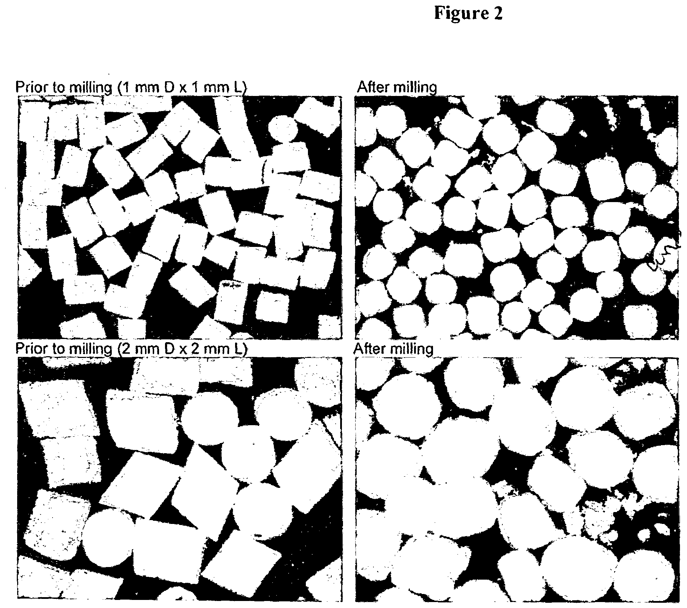

Pellets from sub-lot C were milled in a Krupps mill in six 10-second bursts. Figure 2 depicts the pellets before and after milling (Figure 2A depicts 1 mm x 1 mm pellets, figure 2B depicts 2 mm x 2 mm pellets).

### Example 5

The formulations of Example 5 were prepared in accordance with the following ingredients of Table 5:

**TABLE 5**

| Ingredient (%) and weight (g) | % | Amt/batch (g) |
|---|---|---|
| Oxycodone HCI | 15.00% | 120.000 |
| Eudragit NE Solids, Oven Dried | 70.00% | 560.000 |
| PEO N10 | 15.00% | 120.000 |
| Total | 100.00% | 800.000 |

The formulations were prepared by the following procedures:

### 1. Blending.

Dried and milled materials from Example 4 were placed into a glass jar in the amounts indicated above and blended for about 1 minute. The blend was then passed through a #8 U.S. mesh screen, returned to the jar, and blended for an additional 1 minute.

### 2. Extrusion.

The blend was extruded on a ZSE Extruder using a 20 x 1 mm die plate according to the following parameters in Table 5A:

**TABLE 5A**

| | | Run 1 | Run 2 | Run 3 | Run 4 | Run 5 |
|---|---|---|---|---|---|---|
| Screw Speed (rpm) | | 50 | 50 | 50 | 50 | 50 |
| Motor Torque (%) | | 3 | 43 | 43 | 43 | 43 |
| Melt Pressure (psi) | | -- | -- | 2000 | 1750 | 1550 |
| Melt Temp (°C) | | 112 | 112 | 112 | 125 | 131 |
| Vacuum (m bar) | | -- | 953 | 953 | 953 | 953 |
| Feed Rate (g/min) | | 24 | 24 | 24 | 24 | 24 |
| TEMP (°C) | Zone 1 | 12.8 | 14 | 14.2 | 18 | 18.6 |
| | Zone 2 | 15 | 16.5 | 16.8 | 17.3 | 17.3 |
| | Zone 3 | 15 | 15 | 15.5 | 15 | 15 |
| | Zone 4 | 15 | 15 | 15 | 15 | 15 |
| | Zone 5 | 50 | 50 | 50 | 50 | 50 |
| | Zone 6 | 75 | 75 | 75 | 75 | 75 |
| | Zone 7 | 100 | 100 | 100 | 100 | 100 |
| | Zone 8 | 100 | 100 | 100 | 100 | 100 |
| | Zone 9 | 100 | 100 | 100 | 100 | 100 |
| | Zone 10 | 100 | 100 | 100 | 100 | 100 |
| | Zone 11 MGA | 106.1 | 105 | 105.4 | 115 | 125 |
| | Zone 12 Die | 105.7 | 105 | 109.4 | 118 | 128 |
| Strand Thickness (mm) | | 1mm | 1mm | 1mm | 1mm | 1mm |

The parameters of this example were similar to those of Example 4 except a lower sheer screw design was used and the pelletization was by machine.

### 3. Pellet Preparation; Dissolution and Tamper Resistance Testing.

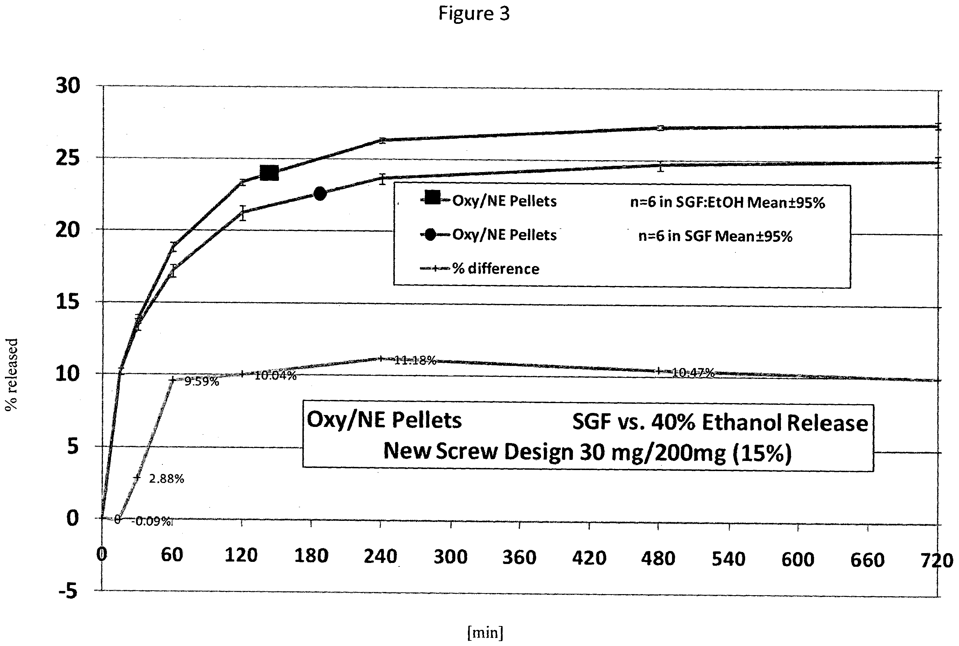

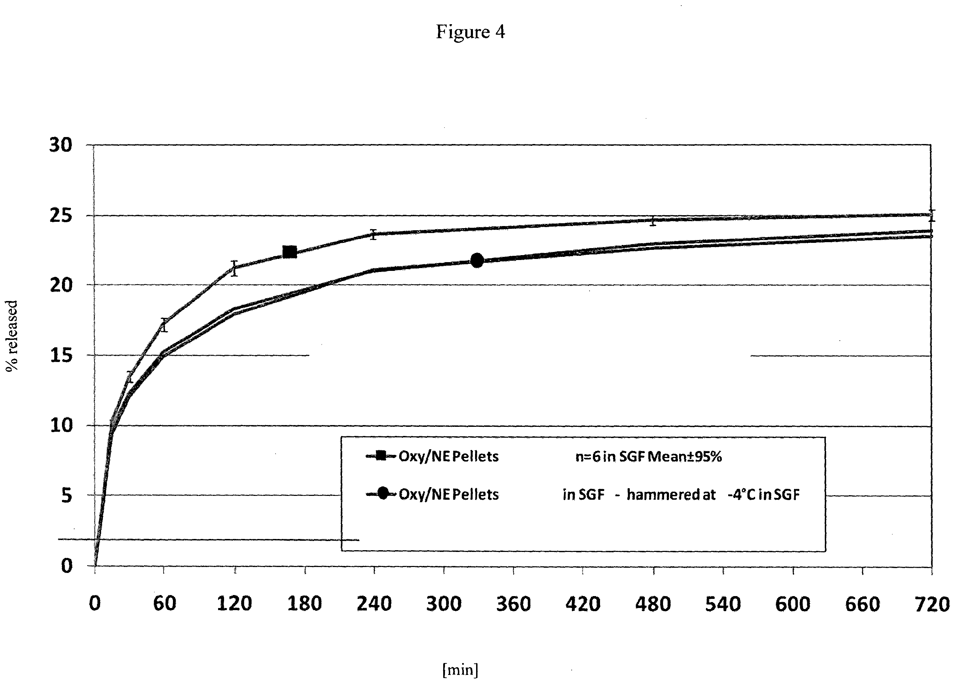

Two extruded strands with approximately a 1mm diameter were fed into a pelletizer machine to produce individual pellets. The pellets were then subject to dissolution and tamper resistance testing. Figure 3 depicts the dissolution of the pellets of this example in (i) simulated gastric fluid (SGF) and (ii) (SGF) and ethanol (EtOH). The dissolution utilized 900 ml SGF with a Basket mesh size of 40 and a height of 25 mm at 100 RPM. Figure 4 depicts the tamper resistance of the pellets of this example, in which pellets in SGF were frozen and subjected to hammering at - 4°C.

The present invention is not to be limited in scope by the specific embodiments disclosed in the examples, which are intended as illustrations of a few aspects of the invention, and any embodiments that are functionally equivalent are within the scope of this invention. Indeed, various modifications of the invention in addition to those shown and described herein will become apparent to those skilled in the art and are intended to fall within the scope of the appended claims.

## Claims

1. An oral solid dosage form comprising a purified neutral acrylic polymer and a prophylactically or therapeutically effective amount of an opioid agonist,
wherein the purified neutral acrylic polymer is obtainable by drying a dispersion comprising a neutral acrylic polymer and comprises 70 - 100 % (w/w) of neutral acrylic polymer, 0 - 10 % (w/w) of water, 0 - 5 % (w/w) of organic solvents, 0 - 2 % (w/w) of emulsifiers and 0 - 5 % (w/w) of further ingredients selected from the group consisting of polymers, poloxamers, bulking agents, release modifying agents, retardants, plasticizers, stabilizers, diluents, fillers, lubricants, binders, granulating aids, colorants, flavorants and glidants,
wherein said neutral acrylic polymer is a poly(meth)acrylate which does not contain free acid groups, amino groups or quaternary ammonium groups,
wherein the oral solid dosage form comprises greater than about 50% (w/w) of the purified neutral acrylic polymer, and
wherein the dosage form further comprises polyethylene oxide and/or a non-ionic triblock copolymer composed of a central hydrophobic chain of polyoxypropylene flanked by two hydrophilic chains of polyoxyethylene.

2. The oral solid dosage form of claim 1, wherein the neutral acrylic polymer is a copolymer or homopolymer of acrylic acid (C₁-C₈) alkyl esters and/or methacrylic acid (C₁-C₈) alkyl esters, preferably a copolymer or homopolymer of acrylic acid (C₁-C₄) alkyl esters and/or methacrylic acid (C₁-C₄) alkyl esters, and more preferably is a copolymer of ethyl acrylate and methyl methacrylate.

3. The oral solid dosage form of any one of claims 1 to 2, wherein the neutral acrylic polymer is a copolymer having the structural formula I 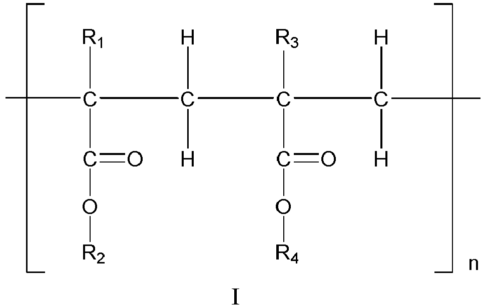
wherein R₁ and R₃ are independently selected from H and methyl,
R₂ and R₄ are independently selected from methyl, ethyl, propyl, butyl, pentyl, hexyl, heptyl or octyl,
and n is selected such that the copolymer has a mean relative molecular mass of at least about 100,000, preferably of at least about 300,000, most preferably of from about 600,000 to about 1,000,000.

4. The oral solid dosage form of any one of claims 1 to 3, wherein the neutral acrylic polymer has a mean relative molecular mass of from about 600,000 to about 1,000,000, preferably of from about 600,000 to 900,000, most preferably of about 660,000, 770,000 or 800,000.

5. The oral solid dosage form of any one of claims 1 to 4, comprising an effective amount of the purified neutral acrylic polymer to provide a controlled release of the opioid agonist, and/or comprising
from about 50% (w/w) to about 80% (w/w) purified neutral acrylic polymer, or
greater than about 60% (w/w) of the purified neutral acrylic polymer, and preferably comprising
from about 50% (w/w) to about 90% (w/w) of purified neutral acrylic polymer, or
from about 60% (w/w) to about 90% (w/w) of purified neutral acrylic polymer.

6. The oral solid dosage form of any one of claims 1 to 5, wherein the oral solid dosage form comprises from about 1% (w/w) to about 50% (w/w) opioid agonist or from about 5% (w/w) to about 40% (w/w) opioid agonist, preferably from about 10% (w/w) to about 30% (w/w) opioid agonist and most preferably from about 15% (w/w) to about 25% (w/w) opioid agonist.

7. The oral solid dosage form of any one of claims 1 to 6, wherein the oral solid dosage form further comprises at least one excipient selected from the group consisting of polymers, poloxamers, bulking agents, release modifying agents, plasticizers, stabilizers, diluents, lubricants, binders, granulating aids, colorants, flavorants, and glidants, and preferably comprises polyethylene oxide.

8. The oral solid dosage form of claim 7, comprising
- from about 50% (w/w) to about 90% (w/w) purified neutral acrylic polymer,
- from about 1% (w/w) to about 50% (w/w) opioid agonist and
- from about 5% (w/w) to about 60% (w/w) polyethylene oxide;
or comprising
- from about 50% (w/w) to about 80% (w/w) purified neutral acrylic polymer,
- from about 5% (w/w) to about 40% (w/w) opioid agonist and
- from about 10% (w/w) to about 50% (w/w) polyethylene oxide;
or comprising
- from about 50% (w/w) to about 70% (w/w) purified neutral acrylic polymer,
- from about 10% (w/w) to about 30% (w/w) opioid agonist and
- from about 15% (w/w) to about 40% (w/w) polyethylene oxide;
or comprising
- from about 50% (w/w) to about 90% (w/w) purified neutral acrylic polymer,
- from about 5% (w/w) to about 50% (w/w) opioid agonist and
- from about 5% (w/w) to about 50% (w/w) polyethylene oxide;
or comprising
- from about 50% (w/w) to about 90% (w/w) purified neutral acrylic polymer,
- from about 5% (w/w) to about 50% (w/w) opioid agonist and
- from about 5% (w/w) to about 40% (w/w) polyethylene oxide.

9. The oral solid dosage form of any one of claims 1 to 8, wherein the oral solid dosage form comprises a matrix formulation comprising the purified neutral acrylic polymer, the opioid agonist and optionally a polyethylene oxide, and wherein the matrix formulation is preferably a controlled release matrix formulation.

10. The oral solid dosage form of any one of claims 1 to 9,
wherein the opioid agonist is selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof, and
wherein the active agent optionally further comprises an opioid antagonist, wherein the opioid antagonist is preferably selected from the group consisting of amiphenazole, naltrexone, methylnaltrexone, naloxone, nalbuphine, nalorphine, nalorphine dinicotinate, nalmefene, nadide, levallorphan, cyclozocine, pharmaceutically acceptable salts thereof and mixtures thereof.

11. A method of preparing an oral solid dosage form in accordance with any one of claims 1 to 10, comprising at least the following steps:
a) drying a dispersion comprising a neutral acrylic polymer to obtain a purified neutral acrylic polymer, wherein said neutral acrylic polymer is a poly(meth)acrylate which does not contain free acid groups, amino groups or quaternary ammonium groups
and wherein preferably the drying is performed by vacuum drying, lyophilization, pan drying, oven drying, freeze drying and/or evaporation;
b) admixing the purified neutral acrylic polymer at least with an active agent to obtain a blend, and simultaneously and/or subsequently
c) further processing the blend to obtain the oral solid dosage form,
wherein in step b), the purified neutral acrylic polymer is, in addition to the active agent, further admixed with polyethylene oxide and/or a non-ionic triblock copolymer composed of a central hydrophobic chain of polyoxypropylene flanked by two hydrophilic chains of polyoxyethylene.

12. The method of claim 11, wherein in step a) the obtained purified neutral acrylic polymer is subsequently milled, preferably in the presence of dry ice.

13. The method of any one of claims 11 to 12, wherein the dispersion is an aqueous dispersion preferably comprising from about 20% (w/w) to about 50% (w/w) neutral acrylic polymer, more preferably comprising from about 30% (w/w) to about 40% (w/w) neutral acrylic polymer.

14. The method of any one of claims 11 to 13, wherein the neutral acrylic polymer is a copolymer having the structural formula I 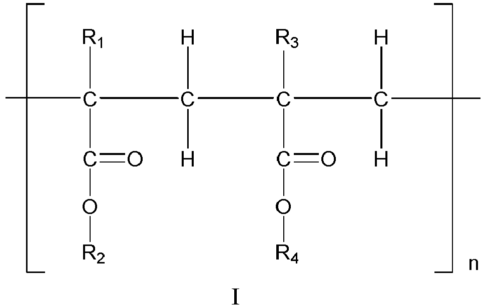
wherein R₁ and R₃ are independently selected from H and methyl,
R₂ and R₄ are independently selected from methyl, ethyl, propyl, butyl, pentyl, hexyl, heptyl or octyl,
and n is selected such that the copolymer has a mean relative molecular mass of at least about 100,000, preferably of at least about 300,000, most preferably of from about 600,000 to about 1,000,000.

15. The method of any one of claims 11 to 14, wherein the purified neutral acrylic polymer obtained in step a) by drying the dispersion comprising the neutral acrylic polymer comprises less than about 20% (w/w) water or less than about 15% (w/w) water, preferably less than about 10% (w/w) water or less than about 5% (w/w) water, more preferably less than about 3% (w/w) water, and most preferably less than about 1% (w/w) water.

16. The method of any one of claims 11 to 15, wherein in step c) the blend is further processed by subjecting it to a direct compression step, preferably by subjecting it to a direct compression step and a subsequent curing step, an extrusion step and preferably a melt-extrusion step, a wet granulation step, a dry granulation step, a hot molding step, or a heat compression step.

17. The method of claim 16, wherein in step c) the blend is further processed by subjecting it to an extrusion step to obtain an extrudate, and wherein the extrudate obtained in step c) is subsequently divided into unitary dosage forms, preferably in the form of a tablet, or into multiparticulates, preferably in the form of pellets or spheres.

18. The method of any one of claims 11 to 17, wherein the active agent is selected from the group consisting of ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha-two-adrenergic agonists, analgesics, antipyretics, anti-inflammatory agents, androgens, local and general anesthetics, antiaddictive agents, antiandrogens, antiarrhythmic agents, antiasthmatic agents, anticholinergic agents, anticholinesterase agents, anticoagulants, antidiabetic agents, antidiarrheal agents, antidiuretic, antiemetic and prokinetic agents, antiepileptic agents, antiestrogens, antifingal agents, antihypertensive agents, antimicrobial agents, antimigraine agents, antimuscarinic agents, antineoplastic agents, antiparasitic agents, antiparkinson's agents, antiplatelet agents, antiprogestins, antischizophrenia agents, antithyroid agents, antitussives, antiviral agents, atypical antidepressants, azaspirodecanediones, barbituates, benzodiazepines, benzothiadiazides, beta-adrenergic agonists, beta-adrenergic antagonists, selective beta-one-adrenergic antagonists, selective beta-two-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, contraceptive agents, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, hematopoietic agents, histamines, histamine antagonists, hormones, 5-hydroxytryptamine antagonists, drugs for the treatment of hyperlipoproteinemia, hypnotics, sedatives, immunosupressive agents, laxatives, methylxanthines, moncamine oxidase inhibitors, neuromuscular blocking agents, organic nitrates, opioid agonists, opioid antagonists, pancreatic enzymes, phenothiazines, progestins, prostaglandins, agents for the treatment of psychiatric disorders, retinoids, sodium channel blockers, agents for spasticity and acute muscle spasms, succinimides, testosterones, thioxanthines, thrombolytic agents, thyroid agents, tricyclic antidepressants, inhibitors of tubular transport of organic compounds, drugs affecting uterine motility, vasodilators, vitamins, and mixtures thereof,
wherein the active agent preferably is an opioid agonist selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone,
papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof, and
wherein the active agent optionally further comprises an opioid antagonist, wherein the opioid antagonist is preferably selected from the group consisting of amiphenazole, naltrexone, methylnaltrexone, naloxone, nalbuphine, nalorphine, nalorphine dinicotinate, nalmefene, nadide, levallorphan, cyclozocine, pharmaceutically acceptable salts thereof and mixtures thereof, or
wherein the active agent is a non-opioid analgesic, wherein the non-opioid analgesic is preferably a non-steroidal anti-inflammatory agent selected from the group consisting of aspirin, celecoxib, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, pharmaceutically acceptable salts thereof and mixtures thereof.

19. The method of preparing an oral solid dosage form according to any one of claims 11 to 18 comprising at least the following steps: (i) mixing in an extruder the purified neutral acrylic polymer, the active agent; and optionally the polyethylene oxide (ii) extruding the mixture as a strand; (iii) cooling the strand; and (iv) dividing the strand into unit doses.

20. An oral solid dosage form according to any one of claims 1 to 10 for use in the treatment of a disease or condition, preferably pain.

## Patentansprüche

1. Orale feste Darreichungsform, umfassend ein gereinigtes neutrales Acrylpolymer und eine prophylaktisch oder therapeutisch wirksame Menge eines Opioid-Agonisten,
wobei das gereinigte neutrale Acrylpolymer durch Trocknen einer Dispersion erhältlich ist, die ein neutrales Acrylpolymer umfasst und 70 - 100 % (Gew./Gew.) neutrales Acrylpolymer, 0 - 10 % (Gew./Gew.) Wasser, 0 - 5 % (Gew./Gew.) organische Lösungsmittel, 0 - 2 % (Gew./Gew.) Emulgatoren und 0 - 5 % (Gew./Gew.) weiterer Bestandteile umfasst, ausgewählt aus der Gruppe bestehend aus Polymeren, Poloxameren, Füllmitteln, freisetzungsmodifizierenden Mitteln, Verzögerungsmitteln, Weichmachern, Stabilisatoren, Verdünnern, Füllstoffen, Schmierstoffen, Bindemitteln, Granulierungshilfen, Farbstoffen, Aroma- und Gleitstoffen,
wobei das neutrale Acrylpolymer ein Poly(meth)acrylat ist, das keine freien Säuregruppen, Aminogruppen oder quartären Ammoniumgruppen enthält,
wobei die orale feste Darreichungsform mehr als etwa 50 % (Gew./Gew.) des gereinigten neutralen Acrylpolymers umfasst, und wobei die Darreichungsform ferner Polyethylenoxid und/oder ein nichtionisches Triblock-Copolymer umfasst, das aus einer zentralen hydrophoben Kette von Polyoxypropylen besteht, die von zwei hydrophilen Ketten von Polyoxyethylen flankiert ist.

2. Orale feste Darreichungsform nach Anspruch 1, wobei das neutrale Acrylpolymer ein Copolymer oder Homopolymer von Acrylsäure (C₁-C₈) alkylestern und/oder Methacrylsäure (C₁-C₈) alkylestern, vorzugsweise ein Copolymer oder Homopolymer von Acrylsäure(C₁-C₄)alkylestern und/oder Methacrylsäure(C₁-C₄)alkylestern ist und mehr bevorzugt ein Copolymer von Ethylacrylat und Methylmethacrylat ist.

3. Orale feste Darreichungsform nach einem der Ansprüche 1 bis 2, wobei das neutrale Acrylpolymer ein Copolymer ist, das die folgende Strukturformel I 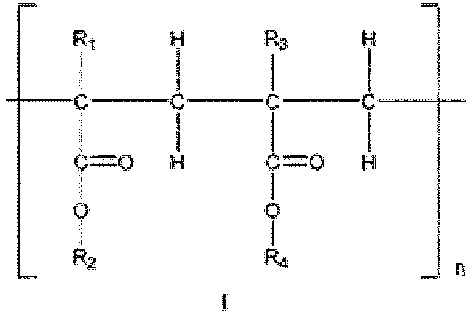
aufweist, wobei R₁ und R₃ unabhängig voneinander aus H und Methyl ausgewählt sind,
R₂ und R₄ unabhängig voneinander aus Methyl, Ethyl, Propyl, Butyl, Pentyl, Hexyl, Heptyl oder Octyl ausgewählt sind
und n so ausgewählt ist, dass das Copolymer eine mittlere relative Molekularmasse von zumindest etwa 100.000, vorzugsweise von zumindest etwa 300.000, am bevorzugtesten von etwa 600.000 bis etwa 1.000.000 aufweist.

4. Orale feste Darreichungsform nach einem der Ansprüche 1 bis 3, wobei das neutrale Acrylpolymer eine mittlere relative Molekularmasse von etwa 600.000 bis etwa 1.000.000, vorzugsweise von etwa 600.000 bis 900.000, am bevorzugtesten von etwa 660.000, 770.000 oder 800.000 aufweist.

5. Orale feste Darreichungsform nach einem der Ansprüche 1 bis 4, umfassend eine wirksame Menge des gereinigten neutralen Acrylpolymers, um eine kontrollierte Freisetzung des Opioid-Agonisten bereitzustellen, und/oder umfassend
von etwa 50 % (Gew./Gew.) bis etwa 80 % (Gew./Gew.) gereinigtes neutrales Acrylpolymer oder
mehr als etwa 60 % (Gew./Gew.) des gereinigten neutralen Acrylpolymers, und
vorzugsweise umfassend
von etwa 50 % (Gew. /Gew.) bis etwa 90 % (Gew. /Gew.) des gereinigten neutralen Acrylpolymers oder
von etwa 60 % (Gew. /Gew.) bis etwa 90 % (Gew. /Gew.) des gereinigten neutralen Acrylpolymers.

6. Orale feste Darreichungsform nach einem der Ansprüche 1 bis 5, wobei die orale feste Darreichungsform von etwa 1 % (Gew. /Gew.) bis etwa 50 % (Gew./Gew.) Opioid-Agonist oder von etwa 5 % (Gew./Gew.) bis etwa 40 % (Gew./Gew.) Opioid-Agonist, vorzugsweise von etwa 10 % (Gew./Gew.) bis etwa 30 % (Gew./Gew.) Opioid-Agonist und am meisten bevorzugt von etwa 15 % (Gew./Gew.) bis etwa 25 % (Gew./Gew.) Opioid-Agonist umfasst.

7. Orale feste Darreichungsform nach einem der Ansprüche 1 bis 6, wobei die orale feste Darreichungsform ferner zumindest einen Hilfsstoff umfasst, ausgewählt aus der Gruppe bestehend aus Polymeren, Poloxameren, Füllmitteln, freisetzungsmodifizierenden Mitteln, Weichmachern, Stabilisatoren, Verdünnern, Schmierstoffen, Bindemitteln, Granulierungshilfen, Farbstoffen, Aroma- und Gleitstoffen, und vorzugsweise Polyethylenoxid umfasst.

8. Orale feste Darreichungsform nach Anspruch 7, umfassend
- von etwa 50 % (Gew./Gew.) bis etwa 90 % (Gew./Gew.) gereinigtes neutrales Acrylpolymer,
- von etwa 1 % (Gew./Gew.) bis etwa 50 % (Gew./Gew.) Opioid-Agonist und
- von etwa 5 % (Gew./Gew.) bis etwa 60 % (Gew./Gew.) Polyethylenoxid;
oder umfassend
- von etwa 50 % (Gew./Gew.) bis etwa 80 % (Gew./Gew.) gereinigtes neutrales Acrylpolymer,
- von etwa 5 % (Gew./Gew.) bis etwa 40 % (Gew./Gew.) Opioid-Agonist und
- von etwa 10 % (Gew./Gew.) bis etwa 50 % (Gew./Gew.) Polyethylenoxid;
oder umfassend
- von etwa 50 % (Gew./Gew.) bis etwa 70 % (Gew./Gew.) gereinigtes neutrales Acrylpolymer,
- von etwa 10 % (Gew./Gew.) bis etwa 30 % (Gew./Gew.) Opioid-Agonist und
- von etwa 15 % (Gew./Gew.) bis etwa 40 % (Gew./Gew.) Polyethylenoxid;
oder umfassend
- von etwa 50 % (Gew./Gew.) bis etwa 90 % (Gew./Gew.) gereinigtes neutrales Acrylpolymer,
- von etwa 5 % (Gew./Gew.) bis etwa 50 % (Gew./Gew.) Opioid-Agonist und
- von etwa 5 % (Gew./Gew.) bis etwa 50 % (Gew./Gew.) Polyethylenoxid;
oder umfassend
- von etwa 50 % (Gew./Gew.) bis etwa 90 % (Gew./Gew.) gereinigtes neutrales Acrylpolymer,
- von etwa 5 % (Gew./Gew.) bis etwa 50 % (Gew./Gew.) Opioid-Agonist und
- von etwa 5 % (Gew./Gew.) bis etwa 40 % (Gew./Gew.) Polyethylenoxid.

9. Orale feste Darreichungsform nach einem der Ansprüche 1 bis 8, wobei die orale feste Darreichungsform eine Matrixformulierung umfasst, die das gereinigte neutrale Acrylpolymer, den Opioid-Agonisten und gegebenenfalls ein Polyethylenoxid umfasst, und wobei die Matrixformulierung vorzugsweise eine Matrixformulierung mit kontrollierter Freisetzung ist.

10. Orale feste Darreichungsform nach einem der Ansprüche 1 bis 9,
wobei der Opioid-Agonist ausgewählt ist aus der Gruppe bestehend aus Alfentanil, Allylprodin, Alphaprodin, Anileridin, Benzylmorphin, Bezitramid, Buprenorphin, Butorphanol, Clonitazen, Codein, Desomorphin, Dextromoramid, Dezocin, Diampromid, Diamorphon, Dihydrocodein, Dihydromorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Fentanyl, Heroin, Hydrocodon, Hydromorphon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Nalbuphin, Narzein, Nicomorphin, Norlevorphanol, Normethadon, Nalorphin, Normorphin, Norpipanon, Opium, Oxycodon, Oxymorphon, Papaveretum, Pentazocin, Phenadoxon, Phenomorphan, Phenazocin, Phenoperidin, Piminodin, Piritramid, Proheptazin, Promedol, Properidin, Propiram, Propoxyphen, Sufentanil, Tilidin, Tramadol, pharmazeutisch verträgliche Salze davon und Mischungen davon, und
wobei das aktive Mittel gegebenenfalls ferner einen Opioid-Antagonisten umfasst, wobei der Opioid-Antagonist vorzugsweise ausgewählt ist aus der Gruppe bestehend aus Amiphenazol, Naltrexon, Methylnaltrexon, Naloxon, Nalbuphin, Nalorphin, Nalorphindinicotinat, Nalmefen, Nadid, Levallorphan, Cyclozocin, pharmazeutisch verträgliche Salze davon und Mischungen davon.

11. Verfahren zum Herstellen einer oralen festen Darreichungsform nach einem der Ansprüche 1 bis 10, umfassend zumindest die folgenden Schritte:
a) Trocknen einer Dispersion, die ein neutrales Acrylpolymer umfasst, um ein gereinigtes neutrales Acrylpolymer zu erhalten, wobei das neutrale Acrylpolymer ein Poly(meth)acrylat ist, das keine freien Säuregruppen, Aminogruppen oder quartären Ammoniumgruppen enthält,
und wobei vorzugsweise das Trocknen durch Vakuumtrocknen, Lyophilisieren, Pfannentrocknen, Ofentrocknen, Gefriertrocknen und/oder Verdampfen durchgeführt wird;
b) Zumischen des gereinigten neutralen Acrylpolymers zumindest mit einem aktiven Mittel, um ein Gemisch zu erhalten, und gleichzeitiges und/oder nachfolgendes
c) Weiterverarbeiten des Gemisches, um die orale feste Darreichungsform zu erhalten,
wobei in Schritt b) das gereinigte neutrale Acrylpolymer zusätzlich zum aktiven Mittel weiterhin mit Polyethylenoxid und/oder einem nichtionischen Triblock-Copolymer zugemischt wird, das aus einer zentralen hydrophoben Kette von Polyoxypropylen besteht, die von zwei hydrophilen Ketten aus Polyoxyethylen flankiert ist.

12. Verfahren nach Anspruch 11, wobei in Schritt a) das erhaltene gereinigte neutrale Acrylpolymer anschließend gemahlen wird, vorzugsweise in Gegenwart von Trockeneis.

13. Verfahren nach einem der Ansprüche 11 bis 12, wobei die Dispersion eine wässrige Dispersion ist, die vorzugsweise von etwa 20 % (Gew./Gew.) bis etwa 50 % (Gew./Gew.) neutrales Acrylpolymer umfasst und bevorzugter von etwa 30 % (Gew./Gew.) bis etwa 40 % (Gew./Gew.) neutrales Acrylpolymer umfasst.

14. Verfahren nach einem der Ansprüche 11 bis 13, wobei das neutrale Acrylpolymer ein Copolymer ist, das die folgende StrukturformelI 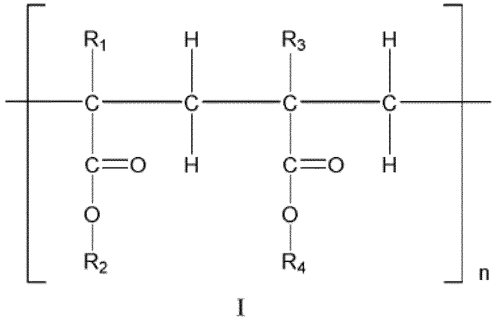
aufweist, wobei R₁ und R₃ unabhängig voneinander aus H und Methyl ausgewählt sind,
R₂ und R₄ unabhängig voneinander aus Methyl, Ethyl, Propyl, Butyl, Pentyl, Hexyl, Heptyl oder Octyl ausgewählt sind
und n so ausgewählt ist, dass das Copolymer eine mittlere relative Molekularmasse von zumindest etwa 100.000, vorzugsweise von zumindest etwa 300.000, am bevorzugtesten von etwa 600.000 bis etwa 1.000.000 aufweist.

15. Verfahren nach einem der Ansprüche 11 bis 14, wobei das gereinigte neutrale Acrylpolymer, das in Schritt a) durch Trocknen der Dispersion umfassend das neutrale Acrylpolymer erhalten wurde, weniger als etwa 20 % (Gew./Gew.) Wasser oder weniger als etwa 15 % (Gew./Gew.) Wasser, vorzugsweise weniger als etwa 10 % (Gew./Gew.) Wasser oder weniger als etwa 5 % (Gew./Gew.) Wasser, mehr bevorzugt weniger als etwa 3 % (Gew./Gew.) Wasser und am bevorzugtesten weniger als etwa 1 % (Gew./Gew.) Wasser umfasst.

16. Verfahren nach einem der Ansprüche 11 bis 15, wobei in Schritt c) das Gemisch weiterverarbeitet wird, indem es einem direkten Kompressionsschritt, vorzugsweise einem direkten Kompressionsschritt und einem nachfolgenden Härtungsschritt, einem Extrusionsschritt und vorzugsweise einem Schmelzextrusionsschritt, einem Nassgranulationsschritt, einem Trockengranulationsschritt, einem Heißformschritt oder einem Heißkompressionsschritt unterzogen wird.

17. Verfahren nach Anspruch 16, wobei in Schritt c) das Gemisch weiterverarbeitet wird, indem es einem Extrusionsschritt unterzogen wird, um ein Extrudat zu erhalten, und wobei das in Schritt c) erhaltene Extrudat anschließend in einheitliche Darreichungsformen, vorzugsweise in Form einer Tablette, oder in Mehrfachteilchen, vorzugsweise in Form von Pellets oder Kugeln, unterteilt wird.

18. Verfahren nach einem der Ansprüche 11 bis 17, wobei das aktive Mittel ausgewählt ist aus der Gruppe bestehend aus ACE-Inhibitoren, adenohypophysäre Hormone, adrenerge Neuronenblocker, adrenerge Steroide, Inhibitoren der Biosynthese von adrenokortikalen Steroiden, alpha-adrenerge Agonisten, alpha-adrenerge Antagonisten, selektive alpha-zwei-adrenerge Agonisten, Analgetika, Antipyretika, entzündungshemmende Mittel, Androgene, lokale und allgemeine Anästhetika, antiaddiktive Mittel, Antiandrogene, antiarrhythmische Mittel, antiasthmatische Mittel, anticholinerge Mittel, Cholinesterase-hemmende Mittel, Antikoagulanzien, antidiabetische Mittel, antidiarrhoische Mittel, antidiuretische, antiemetische und prokinetische Mittel, antiepileptische Mittel, Antiöstrogene, Antimykotika, antihypertensive Mittel, antimikrobielle Mittel, Antimigränemittel, antimuskarinische Mittel, antineoplastische Mittel, antiparasitäre Mittel, Antiparkinsonmittel, Thrombozytenaggregationshemmer, Antiprogestine, Antischizophreniemittel, antithyroide Mittel, Antitussiva, antivirale Mittel, atypische Antidepressiva, Azaspirodecandione, Barbiturate, Benzodiazepine, Benzothiadiazide, beta-adrenerge Agonisten, beta-adrenerge Antagonisten, selektive beta-eins-adrenerge Antagonisten, selektive beta-zwei-adrenerge Agonisten, Gallensalze, Mittel, die das Volumen und die Zusammensetzung der Körperflüssigkeiten beeinflussen, Butyrophenone, Mittel, die die Verkalkung beeinflussen, Kalziumkanalblocker, Herz-Kreislauf-Medikamente, Katecholamine und Sympathomimetika, cholinerge Agonisten, Cholinesterase-Reaktivatoren, Verhütungsmittel, Dermatika, Diphenylbutylpiperidine, Diuretika, Mutterkornalkaloide, Östrogene, Ganglienblocker, Ganglienstimulanzien, Hydantoine, Mittel zur Kontrolle der Magensäure und zur Behandlung von Magengeschwüren, hämatopoetische Mittel, Histamine, Histaminantagonisten, Hormone, 5-Hydroxytryptaminantagonisten, Medikamente zur Behandlung von Hyperlipoproteinämie, Hypnotika, Beruhigungsmittel, Immunsuppressiva, Abführmittel, Methylxanthine, Moncamin-Oxidase-Hemmer, neuromuskuläre Blocker, organische Nitrate, Opioid-Agonisten, Opioid-Antagonisten, Pankreasenzyme, Phenothiazine, Progestine, Prostaglandine, Mittel zur Behandlung von psychiatrischen Störungen, Retinoide, Natriumkanalblocker, Mittel gegen Spastik und akute Muskelkrämpfe, Succinimide, Testosterone, Thioxanthine, Thrombolysemittel, Schilddrüsenmittel, trizyklische Antidepressiva, Hemmer des tubulären Transports organischer Verbindungen, Medikamente, die die Gebärmutterbeweglichkeit beeinflussen, Vasodilatatoren, Vitamine und Mischungen davon, wobei das aktive Mittel vorzugsweise ein Opioid-Agonist ist, ausgewählt aus der Gruppe bestehend aus Alfentanil, Allylprodin, Alphaprodin, Anileridin, Benzylmorphin, Bezitramid, Buprenorphin, Butorphanol, Clonitazen, Codein, Desomorphin, Dextromoramid, Dezocin, Diampromid, Diamorphon, Dihydrocodein, Dihydromorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Fentanyl, Heroin, Hydrocodon, Hydromorphon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Nalbuphin, Narzein, Nicomorphin, Norlevorphanol, Normethadon, Nalorphin, Normorphin, Norpipanon, Opium, Oxycodon, Oxymorphon, Papaveretum, Pentazocin, Phenadoxon, Phenomorphan, Phenazocin, Phenoperidin, Piminodin, Piritramid, Proheptazin, Promedol, Properidin, Propiram, Propoxyphen, Sufentanil, Tilidin, Tramadol, pharmazeutisch verträgliche Salze davon und Mischungen davon, und
wobei das aktive Mittel gegebenenfalls ferner einen Opioid-Antagonisten umfasst, wobei der Opioid-Antagonist vorzugsweise ausgewählt ist aus der Gruppe bestehend aus Amiphenazol, Naltrexon, Methylnaltrexon, Naloxon, Nalbuphin, Nalorphin, Nalorphindinicotinat, Nalmefen, Nadid, Levallorphan, Cyclozocin, pharmazeutisch verträgliche Salze davon und Mischungen davon, oder
wobei das aktive Mittel ein nichtopioides Analgetikum ist, wobei das nichtopioide Analgetikum vorzugsweise ein nichtsteroidales entzündungshemmendes Mittel ist, ausgewählt aus der Gruppe bestehend aus Aspirin, Celecoxib, Ibuprofen, Diclofenac, Naproxen, Benoxaprofen, Flurbiprofen, Fenoprofen, Flubufen, Ketoprofen, Indoprofen, Piroprofen, Carprofen, Oxaprozin, Pramoprofen, Muroprofen, Trioxaprofen, Suprofen, Aminoprofen, Tiaprofensäure, Fluprofen, Bucloxinsäure, Indomethacin, Sulindac, Tolmetin, Zomepirac, Tiopinac, Zidometacin, Acemetacin, Fentiazac, Clidanac, Oxpinac, Mefenaminsäure, Meclofenaminsäure, Flufenaminsäure, Niflumensäure, Tolfenaminsäure, Diflurisal, Flufenisal, Piroxicam, Sudoxicam, Isoxicam, pharmazeutisch verträgliche Salze davon und Mischungen davon.

19. Verfahren zum Herstellen einer oralen festen Darreichungsform nach einem der Ansprüche 11 bis 18, umfassend zumindest die folgenden Schritte: (i) Mischen des gereinigten neutralen Acrylpolymers, des aktiven Mittels; und gegebenenfalls des Polyethylenoxids in einem Extruder; (ii) Extrudieren der Mischung als Strang; (iii) Kühlen des Strangs; und (iv) Unterteilen des Strangs in Einheitsdosen.

20. Orale feste Darreichungsform nach einem der Ansprüche 1 bis 10 zum Verwenden bei der Behandlung einer Krankheit oder eines Zustands, vorzugsweise von Schmerzen.

## Revendications

1. Forme posologique orale solide comprenant un polymère acrylique neutre purifié et une quantité efficace au plan prophylactique ou thérapeutique d'un agoniste opioïde,
dans laquelle le polymère acrylique neutre purifié peut être obtenu par séchage d'une dispersion comprenant un polymère acrylique neutre et comprend entre 70 et 100 % (p/p) de polymère acrylique neutre, entre 0 et 10 % (p/p) d'eau, entre 0 et 5 % de solvants organiques, entre 0 et 2 % (p/p) d'émulsifiants et entre 0 et 5 % (p/p) d'autres ingrédients choisis dans le groupe consistant en polymères, poloxamères, agents de charge, agents modifiant la libération, agents de ralentissement, plastifiants, stabilisants, diluants, charges, lubrifiants, liants, auxiliaires de granulation, colorants, agents aromatisants et agents de glissement,
dans laquelle ledit polymère acrylique neutre est un poly(méth)acrylate qui ne contient pas de groupe acide libre, ni de groupe amino, ni de groupe ammonium quaternaire, laquelle forme posologique orale solide comprend plus d'environ 50 % (p/p) du polymère acrylique neutre purifié, et
laquelle forme posologique comprend en outre un poly(oxyde d'éthylène) et/ou un copolymère à trois blocs non ionique composé d'une chaîne hydrophobe centrale de polyoxypropylène flanquée de deux chaînes hydrophiles de polyoxyéthylène.

2. Forme posologique orale solide selon la revendication 1, dans laquelle le polymère acrylique neutre est un copolymère ou un homopolymère d'esters alkyliques en C₁-C₈ d'acide acrylique et/ou d'esters alkyliques en C₁-C₈ d'acide méthacrylique, de préférence un copolymère ou un homopolymère d'esters alkyliques en C₁-C₄ d'acide acrylique et/ou d'esters alkyliques en C₁-C₄ d'acide méthacrylique, et mieux encore est un copolymère d'acrylate d'éthyle et de méthacrylate de méthyle.

3. Forme posologique orale solide selon l'une quelconque des revendications 1 et 2, dans laquelle le polymère acrylique neutre est un copolymère ayant la formule structurale I 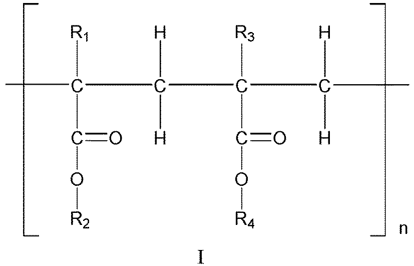
dans laquelle R₁ et R₃ sont indépendamment choisis parmi H et un groupe méthyle,
R₂ et R₄ sont indépendamment choisis parmi les groupes méthyle, éthyle, propyle, butyle, pentyle, hexyle, heptyle ou octyle,
et n est choisi de sorte que le copolymère a une masse moléculaire relative moyenne d'au moins environ 100 000, de préférence d'au moins environ 300 000, mieux encore comprise entre environ 600 000 et environ 1 000 000.

4. Forme posologique orale solide selon l'une quelconque des revendications 1 à 3, dans laquelle le polymère acrylique neutre a une masse moléculaire relative moyenne comprise entre environ 600 000 et 1 000 000, de préférence entre environ 600 000 et 900 000, mieux encore égale à environ 660 000, 770 000 ou 800 000.

5. Forme posologique orale solide selon l'une quelconque des revendications 1 à 4, comprenant une quantité efficace du polymère acrylique neutre purifié pour permettre une libération contrôlée de l'agoniste opioïde, et/ou comprenant d'environ 50 % (p/p) à environ 80 % (p/p) de polymère acrylique neutre purifié, ou plus d'environ 60 % (p/p) de polymère acrylique neutre purifié, et comprenant de préférence d'environ 50 % (p/p) à environ 90 % (p/p) de polymère acrylique neutre purifié, ou
d'environ 60 % (p/p) à environ 90 % (p/p) de polymère acrylique neutre purifié.

6. Forme posologique orale solide selon l'une quelconque des revendications 1 à 5, laquelle forme posologique orale solide comprend d'environ 1 % (p/p) à environ 50 % (p/p) d'agoniste opioïde, ou d'environ 5 % (p/p) à environ 40 % (p/p) d'agoniste opioïde, de préférence d'environ 10 % (p/p) à environ 30 % (p/p) d'agoniste opioïde, et mieux encore d'environ 15 % (p/p) à environ 25 % (p/p) d'agoniste opioïde.

7. Forme posologique orale solide selon l'une quelconque des revendications 1 à 6, laquelle forme posologique orale solide comprend en outre au moins un excipient choisi dans le groupe consistant en polymères, poloxamères, agents de charge, agents modifiant la libération, plastifiants, stabilisants, diluants, lubrifiants, liants, auxiliaires de granulation, colorants, agents aromatisants et agents de glissement, et comprend de préférence du poly(oxyde d'éthylène).

8. Forme posologique orale solide selon la revendication 7, comprenant
- d'environ 50 % (p/p) à environ 90 % (p/p) de polymère acrylique neutre purifié,
- d'environ 1 % (p/p) à environ 50 % (p/p) d'agoniste opioïde, et
- d'environ 5 % (p/p) à environ 60 % (p/p) de poly(oxyde d'éthylène) ;
ou comprenant
- d'environ 50 % (p/p) à environ 80 % (p/p) de polymère acrylique neutre purifié,
- d'environ 5 % (p/p) à environ 40 % (p/p) d'agoniste opioïde, et
- d'environ 10 % (p/p) à environ 50 % (p/p) de poly(oxyde d'éthylène) ;
ou comprenant
- d'environ 50 % (p/p) à environ 70 % (p/p) de polymère acrylique neutre purifié,
- d'environ 10 % (p/p) à environ 30 % (p/p) d'agoniste opioïde, et
- d'environ 15 % (p/p) à environ 40 % (p/p) de poly(oxyde d'éthylène) ;
ou comprenant
- d'environ 50 % (p/p) à environ 90 % (p/p) de polymère acrylique neutre purifié,
- d'environ 5 % (p/p) à environ 50 % (p/p) d'agoniste opioïde, et
- d'environ 5 % (p/p) à environ 50 % (p/p) de poly(oxyde d'éthylène) ;
ou comprenant
- d'environ 50 % (p/p) à environ 90 % (p/p) de polymère acrylique neutre purifié,
- d'environ 5 % (p/p) à environ 50 % (p/p) d'agoniste opioïde, et
- d'environ 5 % (p/p) à environ 40 % (p/p) de poly(oxyde d'éthylène).

9. Forme posologique orale solide selon l'une quelconque des revendications 1 à 8, laquelle forme posologique orale solide comprend une formulation de matrice comprenant le polymère acrylique neutre purifié, l'agoniste d'opioïde et éventuellement un poly(oxyde d'éthylène), et dans laquelle la formulation de matrice est de préférence une formulation de matrice à libération contrôlée.

10. Forme posologique orale solide selon l'une quelconque des revendications 1 à 9,
dans laquelle l'agoniste opioïde est choisi dans le groupe consistant en alfentanil, allylprodine, alphaprodine, aniléridine, benzylmorphine, bézitramide, buprénorphine, butorphanol, clonitazène, codéine, désomorphine, dextromoramide, dézocine, diampromide, diamorphone, dihydrocodéine, dihydromorphine, diménoxadol, diméphéptanol, diméthylthiambutène, butyrate de dioxaphétyle, dipipanone, eptazocine, éthoheptazine, éthylméthylthiambutène, éthylmorphine, étonitazène, fentanyl, héroïne, hydrocodone, hydromorphone, hydroxypéthidine, isométhadone, cétobémidone, lévorphanol, lévophénacylmorphane, lofentanil, mépéridine, meptazinol, métazocine, méthadone, métopon, morphine, myrophine, nalbuphine, narcéine, nicomorphine, norlévorphanol, norméthadone, nalorphine, normorphine, norpipanone, opium, oxycodone, oxymorphone, papavérétum, pentazocine, phénadoxone, phénomorphane, phénazocine, phénopéridine, piminodine, piritramide, proheptazine, promédol, propéridine, propiram, propoxyphène, sufentanil, tilidine, tramadol, des sels pharmaceutiquement acceptables de ceux-ci et des mélanges de ceux-ci, en
dans laquelle l'agent actif comprend éventuellement en outre un antagoniste opioïde, l'antagoniste opioïde étant de préférence choisi dans le groupe consistant en amiphénazole, naltrexone, méthylnaltrexone, naloxone, nalbuphine, nalorphine, dinicotinate de nalorphine, nalméfène, nadide, levallorphane, cyclozocine, des sels pharmaceutiquement acceptables de ceux-ci et des mélanges de ceux-ci.

11. Procédé de préparation d'une forme posologique orale solide selon l'une quelconque des revendications 1 à 10, comprenant au moins les étapes suivantes :
a) séchage d'une dispersion comprenant un polymère acrylique neutre pour obtenir un polymère acrylique neutre purifié, ledit polymère acrylique neutre étant un poly(méth)acrylate qui ne contient pas de groupe acide libre, ni de groupe amino, ni de groupe ammonium quaternaire,
et ledit séchage étant de préférence effectué par séchage sous vide, lyophilisation, séchage en bac, séchage au four, cryodessiccation et/ou évaporation ;
b) mélange du polymère acrylique neutre purifié au moins avec un agent actif pour obtenir un mélange, et simultanément et/ou ensuite
c) traitement consécutif du mélange pour obtenir la forme posologique orale solide,
dans lequel, dans l'étape b), le polymère acrylique neutre purifié est, en plus de l'agent actif, mélangé en outre avec du poly(oxyde d'éthylène) et/ou un copolymère à trois blocs non ionique composé d'une chaîne hydrophobe centrale de polyoxypropylène flanquée de deux chaînes hydrophiles de polyoxyéthylène.

12. Procédé selon la revendication 11, dans lequel, dans l'étape a), le polymère acrylique neutre purifié obtenu est ensuite broyé, de préférence en présence de glace sèche.

13. Procédé selon l'une quelconque des revendications 11 et 12, dans lequel la dispersion est une dispersion aqueuse, comprenant de préférence d'environ 20 % (p/p) à environ 50 % (p/p) de polymère acrylique neutre, mieux encore d'environ 30 % (p/p) à environ 40 % (p/p) de polymère acrylique neutre.

14. Procédé selon l'une quelconque des revendications 11 à 13, dans lequel le polymère acrylique neutre est un copolymère ayant la formule structurale I 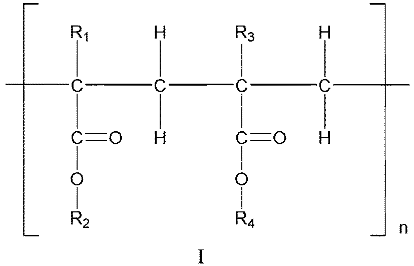
dans laquelle R₁ et R₃ sont indépendamment choisis parmi H et un groupe méthyle,
R₂ et R₄ sont indépendamment choisis parmi les groupes méthyle, éthyle, propyle, butyle, pentyle, hexyle, heptyle ou octyle,
et n est choisi de sorte que le copolymère a une masse moléculaire relative moyenne d'au moins environ 100 000, de préférence d'au moins environ 300 000, mieux encore comprise entre environ 600 000 et environ 1 000 000.

15. Procédé selon l'une quelconque des revendications 11 à 14, dans lequel le polymère acrylique neutre purifié obtenu dans l'étape a) par séchage de la dispersion comprenant le polymère acrylique neutre comprend moins d'environ 20 % (p/p) d'eau ou moins d'environ 15 % (p/p) d'eau, de préférence moins d'environ 10 % (p/p) d'eau ou moins d'environ 5 % (p/p) d'eau, mieux encore moins d'environ 3 % (p/p) d'eau et mieux encore moins d'environ 1 % (p/p) d'eau.

16. Procédé selon l'une quelconque des revendications 11 à 15, dans lequel, dans l'étape c), le mélange est en outre traité en le soumettant à une étape de compression directe, de préférence en le soumettant à une étape de compression directe et ensuite à une étape de durcissement, à une étape d'extrusion et de préférence à une étape d'extrusion en fusion, une étape de granulation en conditions humides, une étape de granulation à sec ou une étape de compression à chaud.

17. Procédé selon la revendication 16, dans lequel, dans l'étape c), le mélange est en outre traité en le soumettant à une étape d'extrusion pour obtenir un extrudat, et dans lequel l'extrudat obtenu dans l'étape c) est ensuite divisé en formes posologiques unitaires, de préférence sous la forme d'un comprimé, ou en formes multiparticulaires, de préférence sous la forme de pastilles ou de sphères.

18. Procédé selon l'une quelconque des revendications 11 à 17, dans lequel l'agent actif est choisi dans le groupe consistant en inhibiteurs d'ACE, hormones adénohypophosaires, agents bloquant les neurones adrénergiques, stéroïdes corticosurrénaux, inhibiteurs de la biosynthèse des stéroïdes corticosurrénaux, agonistes alpha-adrénergiques, antagonistes alpha-adrénergiques, agonistes alpha-2-adrénergiques sélectifs, analgésiques, antipyrétiques, agents anti-inflammatoires, androgènes, anesthésiques locaux et généraux, agents antiaddiction, antiandrogènes, agents antiarrhythmiques, agents antiasthmatiques, agents anticholinergiques, agents anticholinestérase, anticoagulants, agents antidiabétiques, antidiarrhéiques, agents antidiurétiques, agents antiémétiques et prokinétiques, agents antiépileptiques, antioestrogènes, agents antifongiques, agents antihypertenseurs, agents antimicrobiens, agents antimigraineux, agents antimuscariniques, agents antinéoplasiques, agents antiparasitaires, agents antiparkinsoniens, agents antiplaquettes, agents antiprogestérone, agents antischizophréniques, agents antithyroïdiens, antitussifs, agents antiviraux, antidépresseurs atypiques, azaspirodécanediones, barbituriques, benzodiazépines, benzothiadiazides, agonistes bêta-adrénergique, antagonistes bêta-adrénergiques, antagonistes bêta-1-adrénergiques sélectifs, agonistes bêta-2-adrénergiques sélectifs, sels biliaires, agents affectant le volume et la composition des fluides corporels, butyrophénones, agents affectant la calcification, agents bloquant les canaux calciques, médicaments cardiovasculaires, catécholamines et médicaments sympathomimétiques, agonistes cholinergiques, réactivateurs de la cholinestérase, agents contraceptifs, agents dermatologiques, diphénylbutylpipéridines, diurétiques, alcaloïdes de l'ergot, oestrogènes, ganglioplégiques, agents stimulants ganglionnaires, hydantoïnes, agents de régulation de l'acidité gastrique et de traitement d'ulcères gastroduodénaux, agents hématopoïétiques, histamines, antagonistes d'histamine, hormones, antagonistes de 5-hydroxytryptamine, médicaments pour le traitement de l'hyperlipoprotéinémie, hypnotiques, sédatifs, agents immunodépresseurs, laxatifs, méthylxanthines, inhibiteurs de monoamine oxydase, agents bloquants neuromusculaires, nitrates organiques, agonistes opioïdes, antagonistes opioïdes, enzymes pancréatiques, phénothiazines, progestines, prostaglandines, agents pour le traitement de troubles psychiatriques, rétinoïdes, agents bloquant les canaux sodiques, agents pour la spasticité et les spasmes musculaires aigus, succinimides, testostérones, thioxanthines, agents thrombolytiques, agents thyroïdiens, antidépresseurs tricycliques, inhibiteurs du transport tubulaire des composés organiques, médicaments affectant la motilité utérine, vasodilatateurs, vitamines et mélanges de ceux-ci,
dans lequel l'agent actif est de préférence un agoniste opioïde choisi dans le groupe consistant en alfentanil, allylprodine, alphaprodine, aniléridine, benzylmorphine, bézitramide, buprénorphine, butorphanol, clonitazène, codéine, désomorphine, dextromoramide, dézocine, diampromide, diamorphone, dihydrocodéine, dihydromorphine, diménoxadol, diméphéptanol, diméthylthiambutène, butyrate de dioxaphétyle, dipipanone, eptazocine, éthoheptazine, éthylméthylthiambutène, éthylmorphine, étonitazène, fentanyl, héroïne, hydrocodone, hydromorphone, hydroxypéthidine, isométhadone, cétobémidone, lévorphanol, lévophénacylmorphane, lofentanil, mépéridine, meptazinol, métazocine, méthadone, métopon, morphine, myrophine, nalbuphine, narcéine, nicomorphine, norlévorphanol, norméthadone, nalorphine, normorphine, norpipanone, opium, oxycodone, oxymorphone, papavérétum, pentazocine, phénadoxone, phénomorphane, phénazocine, phénopéridine, piminodine, piritramide, proheptazine, promédol, propéridine, propiram, propoxyphène, sufentanil, tilidine, tramadol, des sels pharmaceutiquement acceptables de ceux-ci et des mélanges de ceux-ci,et
dans lequel l'agent actif comprend éventuellement en outre un antagoniste opioïde, l'antagoniste opioïde étant de préférence choisi dans le groupe consistant en amiphénazole, naltrexone, méthylnaltrexone, naloxone, nalbuphine, nalorphine, dinicotinate de nalorphine, nalméfène, nadide, levallorphane, cyclozocine, des sels pharmaceutiquement acceptables de ceux-ci et des mélanges de ceux-ci, ou
dans lequel l'agent actif est de préférence un analgésique non opioïde, l'analgésique non opioïde étant de préférence un agent anti-inflammatoire non stéroïdien choisi dans le groupe consistant en aspirine, célécoxib, ibuprofène, diclofénac, naproxène, bénoxaprofène, flurbiprofène, fénoprofène, flubufène, kétoprofène, indoprofène, piroprofène, carprofène, oxaprozine, pramoprofène, muroprofène, trioxaprofène, suprofène, aminoprofène, acide tiaprofénique, fluprofène, acide bucloxique, indométhacine, sulindac, tolmétine, zomépirac, tiopinac, zidométacine, acémétacine, fentiazac, clidanac, oxpinac, acide méfénamique, acide méclofénamique, acide flufénamique, acide niflumique, acide tolfénamique, diflurisal, flufénisal, piroxicam, sudoxicam, isoxicam, des sels pharmaceutiquement acceptables de ceux-ci et des mélanges de ceux-ci.

19. Procédé de préparation d'une forme posologique orale solide selon l'une quelconque des revendications 11 à 18, comprenant au moins les étapes suivantes : (i) mélange, dans une extrudeuse, du polymère acrylique neutre, de l'agent actif et éventuellement du poly(oxyde d'éthylène), (ii) extrusion du mélange sous forme de brin, (iii) refroidissement du brin, et (iv) division du brin en doses unitaires.

20. Forme posologique orale solide selon l'une quelconque des revendications 1 à 10, destinée à être utilisée dans le traitement d'une maladie ou d'une affection, de préférence une douleur.
